# EUROPEAN PATENT SPECIFICATION

(11) **EP 1 597 262 B1**
(45) Date of publication and mention of the grant of the patent: **11.11.2009**
(21) Application number: 04708731.7
(22) Date of filing: 06.02.2004
(51) Int. Cl.: C07D 491/04, C07D 207/26, A61P 35/00

(54) **SUBSTITUTED HETEROCYCLES**
SUBSTITUIERTE HETEROZYKLEN
HETEROCYCLES SUBSTITUES

(30) Priority: 14.02.2003 EP 03003495; 02.04.2003 EP 03007594
(43) Date of publication of application: 23.11.2005
(73) Proprietor: InterMed Discovery GmbH, 44227 Dortmund (DE)
(72) Inventor: STADLER, Marc, 42115 Wuppertal (DE); SEIP, Stephan, 58332 Schwelm (DE); MÜLLER, Hartwig, 42553 Velbert (DE); MAYER-BARTSCHMID, Anke, 42489 Wülfrath (DE); BRÜNING, Michael-Alexander, 42327 Wuppertal (DE); BENET-BUCHHOLZ, Jordi, E 08397 Pinela de Mar (ES); TOGAME, Hiroko, Osaka-shi, Osaka-fu, 530-0041 (JP); DODO, Reiko, Hyogo-ken, 662-0912 (JP); REINEMER, Peter, 82152 Martinsried (DE); BACON, Kevin, La Jolla, 92037 (CA); FUCHIKAMI, Kinji, Minato-ku, Tokyo-to, 108-0072 (JP); MATSUKAWA, Satoko, Yao-shi, 581-0036 (JP); URBAHNS, Klaus, S-223 51 Lund (SE)
(74) Representative: Kutzenberger, Helga
(86) International application number: PCT/EP2004/001097
(87) International publication number: WO 2004/071382

(56) References cited:
- WO-A-02/47610
- WO-A-96/32105

## Description

The present invention relates to substituted heterocycles, processes for their preparation, and their use in medicaments, especially for the treatment of inflammatory disease, i.e. asthma, or cancer.

The multicatalytic proteinase or proteasome is a highly conserved cellular structure that is responsible for the ATP-dependent proteolysis of most cellular proteins. The 20S (700-kDa) proteasome contains at least five distinct proteolytic activities that have a new type of mechanism involving a threonine residue at the active site (Coux, O., Tanaka, K. and Goldberg, A. 1996 Ann. Rev. Biochem. 65:801-47).

Although the 20S proteasome contains the proteolytic core, it cannot degrade proteins in vivo unless it is complexed with a 19S cap at either end of its structure, which itself contains multiple ATPase activities. This larger structure is known as the 26S proteasome and rapidly degrades proteins that have been targeted for degradation by the addition of multiple molecules of the 8.5-kDa polypeptide, ubiquitin.

It is now well established that the proteasome is a major extralysosomal proteolytic system which is involved in the degradative pathways resulting in numerous and diverse cellular functions such as cell division, antigen processing and the degradation of short lived regulatory proteins such as transcription factors, oncogene products and cyclins (reviewed in Ciechanover, A. 1994 Cell 79:13-21). The primary function of the proteasome is to catalyze the proteolysis of proteins into small peptides. However, it has also been demonstrated that the ubiquitin-proteasome pathway can catalyze the regulated proteolytic processing of a large inactive precursor into an active protein. The best documented case of this involves the activation of the transcription factor NF-κB (Palombella, V. J., Rando, O. J., Goldberg, A. L., and Maniatis, T. 1994 Cell 78:773-785). The active form of NF-κB is a heterodimer consisting of a p65 and a p50 subunit. The latter is present in the cytosol of the cell in an inactive precursor form, namely p105, the 105-kDa polypeptide precursor of p50. The proteolytic processing of p105 to generate p50 occurs via the ubiquitin-proteasome pathway. Additionally, processed p50 and p65 is maintained in the cytosol as an inactive complex with the inhibitory protein IκB. Inflammatory signals activate NF-κB by initiating the signalling pathway for the complete degradation of IκB, and also stimulate the processing of p105 into p50. Thus two proteolytic events, both governed by the ubiquitin-proteasome pathway, are required for signal induced activation of NF-kB.

Once activated, NF-κB translocates to the nucleus, where it plays a central role in the regulation of a remarkably diverse set of genes involved in the immune and inflammatory responses (Grilli et al., International Review of Cytology (1993) 143:1-62). For example, NF-κB is required for the expression of a number of genes involved in the inflammatory response, such as TNF-α gene and genes encoding the cell adhesion molecules E-selectin, P-selectin, ICAM, and VCAM (Collins, T., Lab. Invest. (1993) 68:499). NF-κB is also required for the expression of a large number of cytokine genes such as IL-2, IL-6, granulocyte colony stimulating factor, and IFN-β. Inducible nitric oxide synthetase is also under regulatory control of NF-κB. Proteasome inhibitors block IκBα degradation and activation of NF-κB (Palombella et al. WO 95/25533 published Sep. 28, 1995; Traenckner, et al., EMBO J. (1994) 13:5433). Proteasome inhibitors also block TNF-α induced expression of the leukocyte adhesion molecules E-selectin, VCAM-1, and ICAM-1 (Read, et al., Immunity (1995) 2:493).

The fact that the proteasome plays a critical event in the activation of NF-κB could be exploited clinically by the use of inhibitors directed towards proteasome proteolysis. In certain diseases the normal function of active NF-κB can be detrimental to human health as observed in inflammatory responses. Thus inhibitors of NF-κB activation, due to their ability to prevent secretion of various inflammatory molecules such as cell adhesion molecules or cytokines, may have potential utility in the treatment of inflammatory disorders such as inflammatory disorders including, for example, allergy, COPD, airway inflammation and asthma, ARDS (acute respiratory distress syndrome), AIDS, osteo arthritis and rheumatoid arthritis; inflammatory bowel disease, including ulcerative colitis and Crohn's disease; sepsis; transplant rejection and ischemia or reperfusion injury, including stroke and myocardial infarction.. Since activation of NF-κB is also essential for angiogenesis, proteasome inhibitors may have utility in the treatment of the diseases associated with abnormal neovascularization.

p53 was first described as an oncoprotein but has since been shown to be involved in many cellular processes (reviewed by Ko, L. J. and Proves, C. 1996 Genes Dev. 10, 1054-1072). p53 has been shown to induce apoptosis in several haematopoietic cell lines (Oren, M., 1994 Semin. Cancer Biol. 5, 221-227) through the action of many different stimuli including DNA damage, viral infection and the removal of growth factors. However, it is important to note that apoptosis can be induced in a p53-independent manner for example by the action of glucocorticoids. Induction of p53 leads to cell growth arrest in the G1 phase of the cell cycle as well as cell death by apoptosis. Both of these functions allow p53 to control DNA damage thereby reducing the propagation of DNA mutations when cells divide. p53 arrests cells at G1 by inducing the cyclin-dependent kinase inhibitor, p21, which in turn causes an accumulation of the hypophosphorylated form of the retinoblastoma gene product. It is thought that p53 acts as a check point in the cell following DNA damage, it first causes an arrest in cell division and apoptosis. p53 degradation is known to be via the ubiquitin-proteasome pathway and disrupting p53 degradation is a possible mode of inducing apoptosis. Another potential utility of proteasome inhibitors may be in the treatment of diseases that result from abnormal cell proliferation.

It is well documented that the ubiquitin-proteasome pathway is critical for the regulated destruction of cyclins that govern the exit from mitosis and allow cells to progress into the next phase of the cell cycle. Thus inhibiting degradation of cyclins by using proteasome inhibitors causes growth arrest. Therefore another potential utility of proteasome inhibitors is their use in the treatment of diseases that result from an accelerated cell division. These include cancer, cardiovascular diseases such as myocarditis, restenosis following angioplasty, renal diseases such as lupus, polycystic kidney disease, fungal infections, dermatological diseases such as psoriasis, abnormal wound healing, keloids, immunological diseases such as autoimmunity, acute and delayed hypersensitivity, graft versus host disease, transplant rejection and neuroimmunological diseases such as multiple sclerosis and acute disseminated encephalomyelitis.

Several microbial metabolites were found to inhibit the proteasome. For instance, some peptidic compounds have been reported from streptomycetes such as the TMC-96 series (Y. Koguchi et al., J. Antibiot., 1999, 52, 63-65 and J. Antibiot., 2000, 53, 1069-1076) and fungi such as the TMC-95 series (J. Kohno et al., J. Org. Chem., 2000, 65, 990-995) as strong inhibitors of this target. Non-peptidic actinomycete metabolites possessing a beta-lactone moiety or their respective chemical analogues have also been claimed to strongly interact with this target. Among those are the Salinosporamides from marine actinomycete *Salinospora* sp. known from WO 02/47610 and R. Feling et al., Angew. Chem. Int. Ed. Engl. 2003, 42, 355-357 (Salinosporamide A), and lactacystin β-lactones known from WO 96/32105, WO 99/15183 and WO 99/09006.

Salinosporamide E and Salinosporamide G are known from the 10^{th} Internat. Symp. on Marine Natural Prod. in Okinawa 2001 and "Salinosporamide-A" is known form the 50th Annual Congress Society for Medicinal Plant Research in Barcelona, Spain, 8 -12 September 2002.

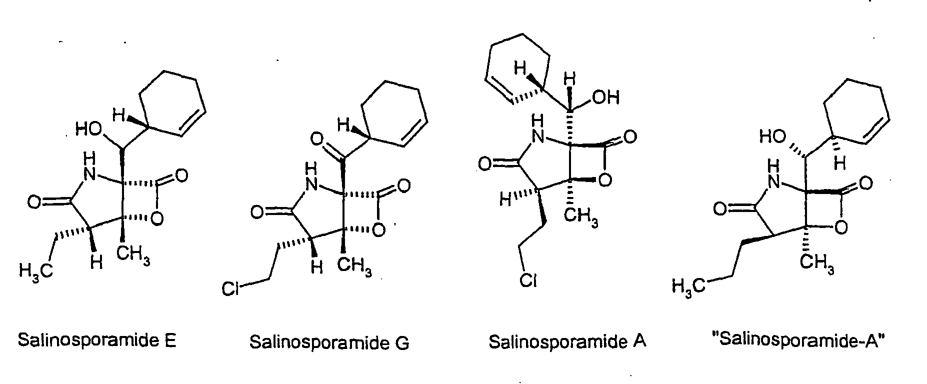

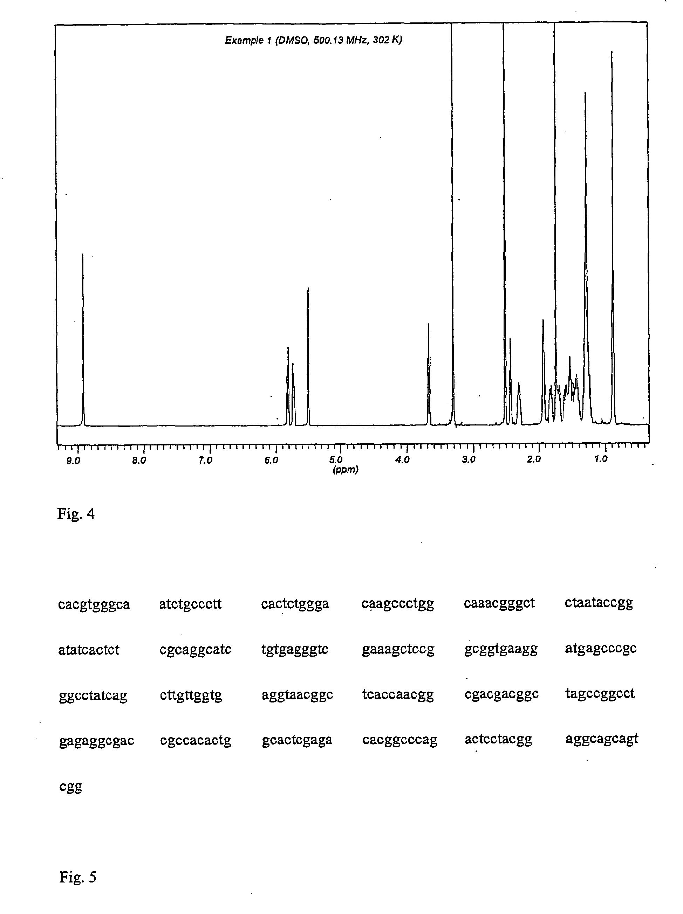

The present invention relates to novel substituted heterocycles which show unprecedented strong inhibition of the proteasome and the isolation of these compounds from the novel Actinomycete JS360 (DSM 15324) of the genus *Streptomyces* with SEQ ID NO: 1 as disclosed in Fig. 5 and the sequence listing.

The present invention relates to compounds of formula 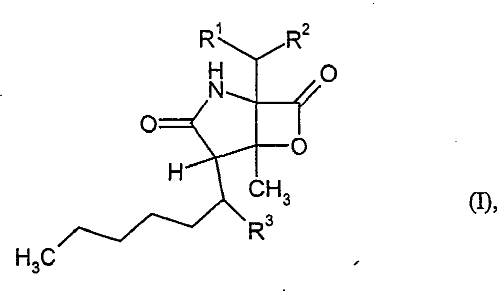 wherein
- R¹: represents hydrogen, hydroxy or methylcarbonyloxy,
- R²: represents cyclohexyl or cyclohex-2-enyl, wherein cyclohexyl can be substituted with 0 to 2 hydroxy groups,
and
- R³: represents hydrogen or hydroxy.

The compounds according to the invention can also be present in the form of their salts, solvates or solvates of the salts.

Depending on their structure, the compounds according to the invention can exist in stereoisomeric forms (enantiomers, diastereomers). The invention therefore relates to the enantiomers or diastereomers and to their respective mixtures. Such mixtures of enantiomers and/or diastereomers can be separated into stereoisomerically unitary constituents in a known manner.

The invention also relates to tautomers of the compounds, depending on the structure of the compounds.

Salts for the purposes of the invention are preferably physiologically acceptable salts of the compounds according to the invention.

Physiologically acceptable salts of the compounds (I) include acid addition salts of mineral acids, carboxylic acids and sulphonic acids, for example salts of hydrochloric acid, hydrobromic acid, sulphuric acid, phosphoric acid, methanesulphonic acid, ethanesulphonic acid, toluenesulphonic acid, benzenesulphonic acid, naphthalenedisulphonic acid, acetic acid, propionic acid, lactic acid, tartaric acid, malic acid, citric acid, fumaric acid, maleic acid and benzoic acid.

Physiologically acceptable salts of the compounds (I) also include salts of customary bases, such as for example and preferably alkali metal salts (for example sodium and potassium salts, alkaline earth metal salts (for example calcium and magnesium salts) and ammonium salts derived from ammonia or organic amines having 1 to 16 carbon atoms, such as illustratively and preferably ethylamine, diethylamine, triethylamine, ethyldiisopropylamine, monoethanolamine, diethanolamine, triethanolamine, dicyclohexylamine, dimethylaminoethanol, procaine, dibenzylamine, N-methylmorpholine, dihydroabietylamine, arginine, lysine, ethylenediamine and methylpiperidine.

Solvates for the purposes of the invention are those forms of the compounds that coordinate with solvent molecules to form a complex in the solid or liquid state. Hydrates are a specific form of solvates, where the coordination is with water.

### Explanation of the figures

- Fig. 1:: Time course of fermentation of strain JS360 in 301 scale.
- Fig. 2:: Scheme for the isolation of examples 1 to 7 from the crude extracts of a fermentation of strain JS 360 in 30 litre scale.
- Fig. 3:: HPLC-chromatograms, HPLC-UV and HPLC-ESI LC-MS spectra of example 1 after preparative HPLC.
- Fig. 4:: Proton spectrum of example 1.
- Fig. 5:: SEQ ID NO: 1: Partial 16S rDNA, a partial sequence of strain JS360/DSM 15324.
- Fig. 6:: Dendrogram showing the relationships between *Salinospora* sp. and JS360. The scale beneath the tree represents the distance between sequences. Units indicate the number of substitution events. *Salinospora* sp. cluster in the upper branch, whereas JS360 and similar sequences cluster in the lower branch.
- Fig. 7:: Ortep-Plot (50%) of example 1 with the numbering of non hydrogen atoms.
- Fig. 8:: Crystal packing of example 1 with view along the a and c axes showing the polar and non polar layers.

In another embodiment, the present invention relates to compounds according to formula (I),
wherein
- R¹: represents hydrogen or hydroxy,
- R²: represents cyclohexyl or cyclohex-2-enyl, wherein cyclohexyl can be substituted with 0 to 2 hydroxy groups,
and
- R³: represents hydrogen or hydroxy.

In another embodiment, the present invention relates to compounds according to formula 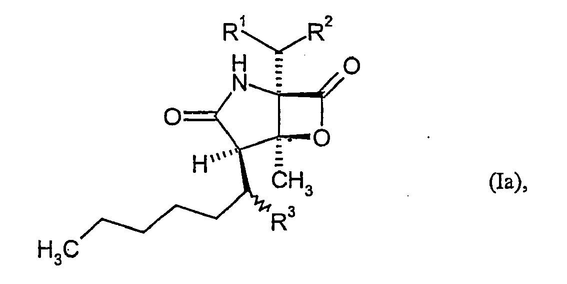 wherein
R¹, R² and R³ have the meaning described above.

In another embodiment, the present invention relates to compounds according to formula (I), such as
(1R,4R,5S)-1-[(S)-(1S)-2-cyclohexen-1-yl(hydroxy)methyl]-4-hexyl-5-methyl-6-oxa-2-azabicyclo[3.2.0]heptane-3,7-dione 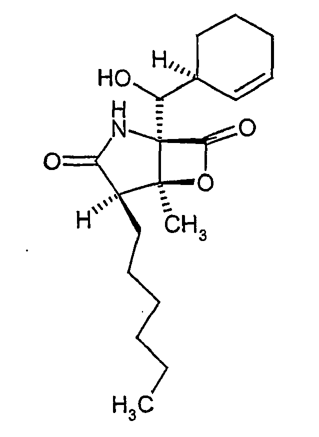 (1R,4R,5S)-1-[(S)-(1S)-2-cyclohexen-1-yl(hydroxy)methyl]-4-[1-hydroxy-hexyl]-5-methyl-6-oxa-2-azabicyclo[3.2.0]heptane-3,7-dione 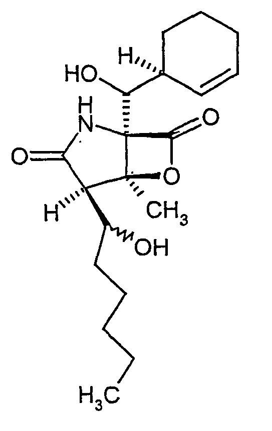 and
(1R,4R,5S)-1-[(1R)-2-cyclohexen-1-ylmethyl]-4-hexyl-5-methyl-6-oxa-2-aza-bicyclo[3.2.0]-heptane-3,7-dione

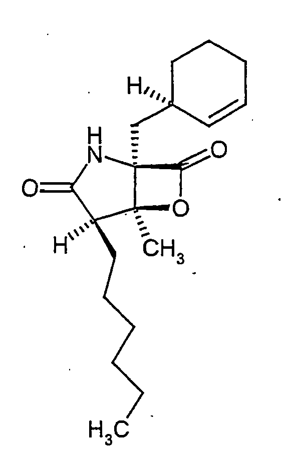

In another embodiment, the present invention relates to compounds according to formula 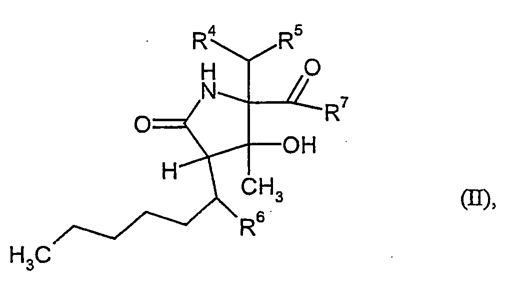 wherein
- R⁴: represents hydrogen or hydroxy,
- R⁵: represents cyclohexyl or cyclohex-2-enyl,
wherein cyclohexyl can be substituted with 0 to 2 hydroxy groups,
- R⁶: represents hydrogen or hydroxy,
and
- R⁷: represents hydroxy or
a substituent of the formula of the group consisting of 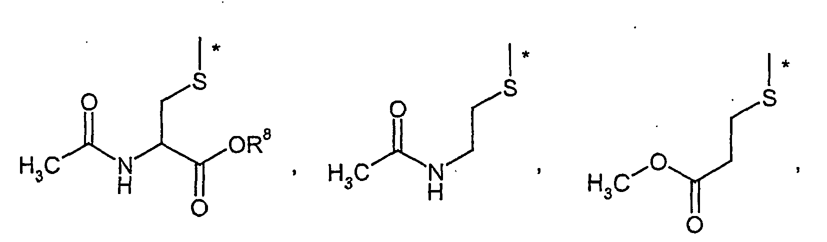 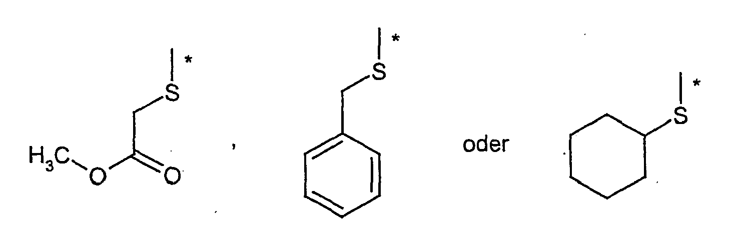 wherein
R⁸ represents hydrogen or methyl, and
* represents the connection position to the molecule.

In another embodiment, the present invention relates to compounds according to formula 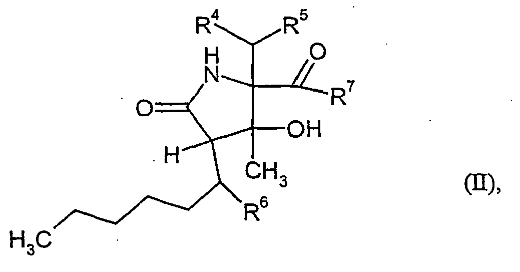 wherein
- R⁴: represents hydrogen or hydroxy,
- R⁵: represents cyclohexyl or cyclohex-2-enyl, wherein cyclohexyl can be substituted with 0 to 2 hydroxy groups,
- R⁶: represents hydrogen or hydroxy,
and
- R⁷: represents hydroxy or a substituent of the formula 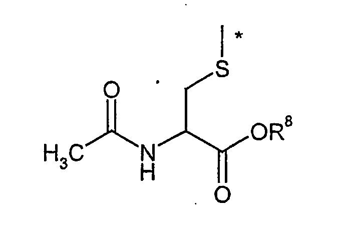
wherein
- R⁸: represents hydrogen or methyl,
and
- *: represents the connection position to the molecule.

In another embodiment, the present invention relates to compounds according to formula 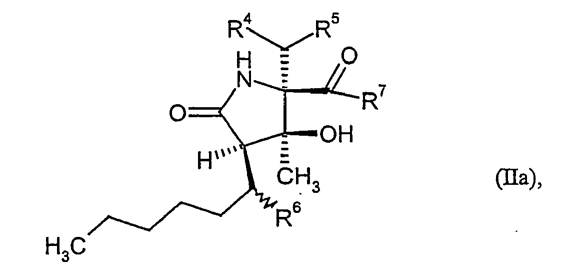 wherein
R⁴, R⁵, R⁶ and R⁷ have the meaning described above.

In another embodiment, the present invention relates to compounds according to formula (II), such as
(3S,4R)-2-[(S)-(1S)-2-cyclohexen-1-yl(hydroxy)methyl]-3-hydroxy-4-[1-hydroxy-hexyl]-3-methyl-5-oxo-D-proline 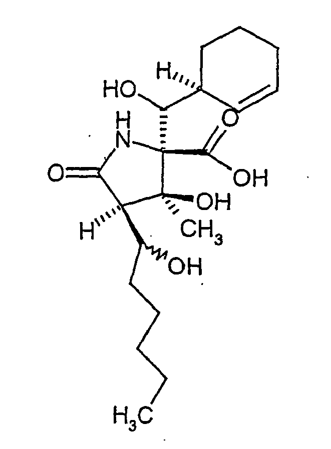 N-acetyl-S-({(2R,3S,4R)-2-[(S)-(1S)-2-cyclohexen-1-yl(hydroxy)methyl]-4-hexyl-3-hydroxy-3-methyl-5-oxo-2-pyrrolidinyl}carbonyl)cysteine 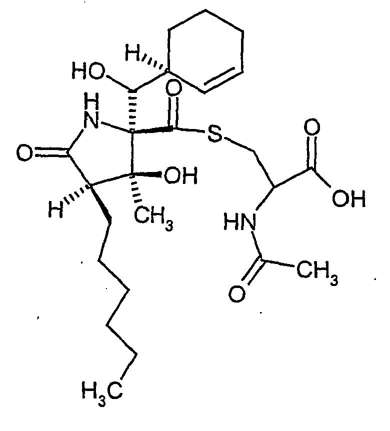 and
methyl-N-acetyl-S-({(2R,3S,4R)-2-[(S)-(1S)-2-cyclohexen-1-yl(hydroxy)methyl]-4-hexyl-3-hydroxy-3-methyl-5-oxo-2-pyrrolidinyl}carbonyl)cysteinate

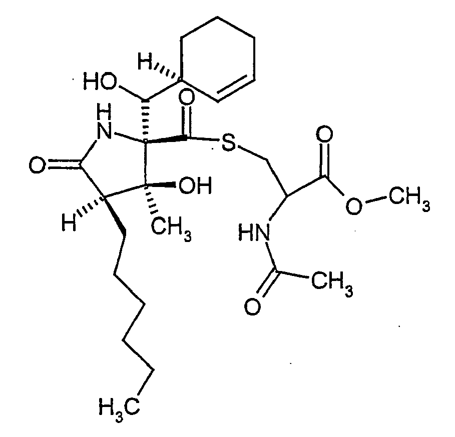

In another embodiment, the present invention relates to an Actinomycete of the genus *Streptomyces* with SEQ ID NO: 1, /Fig. 5 and sequence listing)

In another embodiment, the present invention relates
[A] to a process for synthesizing the compounds of general formula 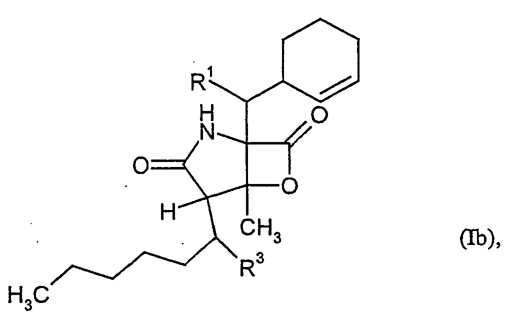 wherein
   R¹ and R³ have the meaning described above,
   characterized in that the compounds are prepared via fermentation and isolation from an Actinomycete JS360 (DSM 15324) of the genus *Streptomyces* with SEQ ID NO: 1,
   or
[B] to a process for synthesizing the compounds of general formula 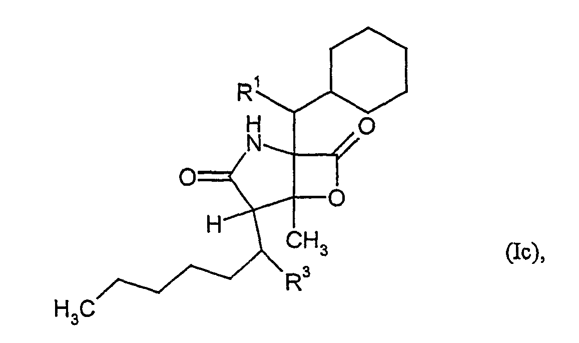 wherein
   R¹ and R³ have the meaning described above,
   characterized in that the compounds are prepared via hydrogenation of the double bond in compounds of the formula (Ib),
   or
[C] to a process for synthesizing the compounds of general formula 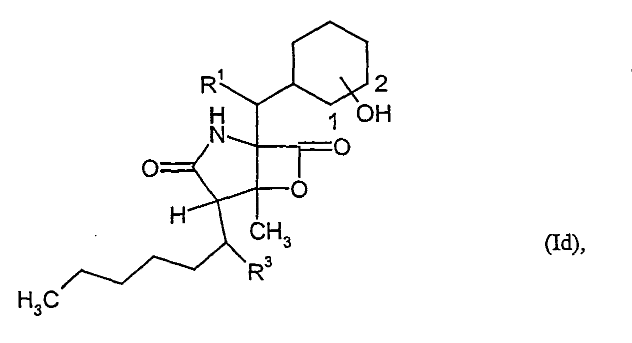 wherein
   R¹ and R³ have the meaning described above, and
   the hydroxy-group is attached onto carbon atom 1 or 2,
   characterized in that the compounds are prepared via hydration of the double bond in compounds of the formula (Ib),
   or
[D] to a process for synthesizing the compounds of general formula 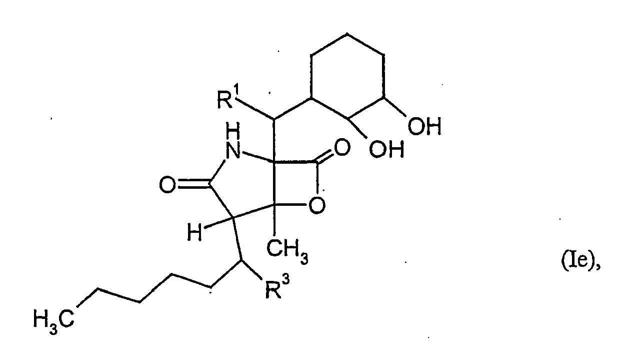 wherein
   R¹ and R³ have the meaning described above,
   characterized in that the compounds are prepared via oxidation of the double bond in compounds of the formula (Ib),
   or
[E] to a process for synthesizing the compounds of general formula 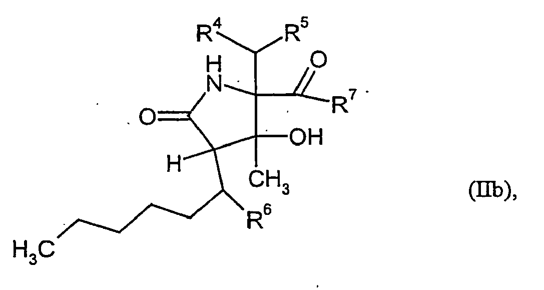 wherein
   R⁴, R⁵ and R⁶ have the meaning described above, and
   R⁷ represents hydroxy or a substituent of the formula 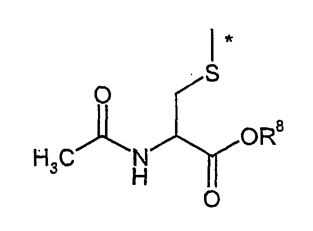 wherein
      R⁸ has the meaning described above,
   characterized in that the compounds are prepared via fermentation and isolation from an Actinomycete of the genus *Streptomyces* with SEQ ID NO: 1,
   or
[F] to a process for synthesizing the compounds of general formula 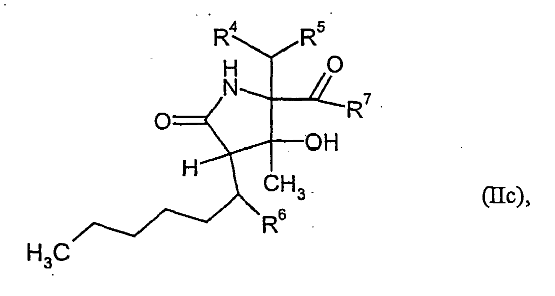 wherein
   R⁴, R⁵ and R⁶ have the meaning described above, and
   R⁷ represents a substituent of the formula of the group consisting of 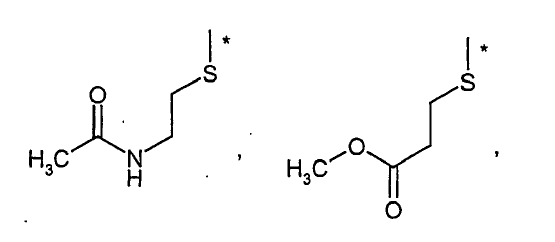 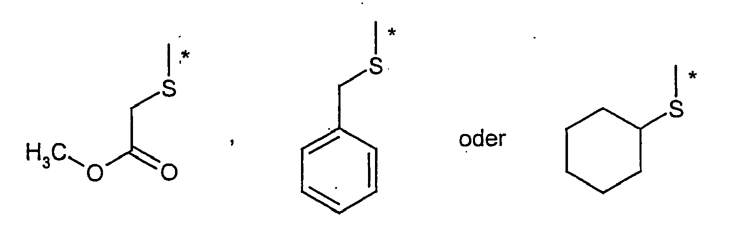
   characterized in that the compounds are prepared via reaction of the compounds of the formula 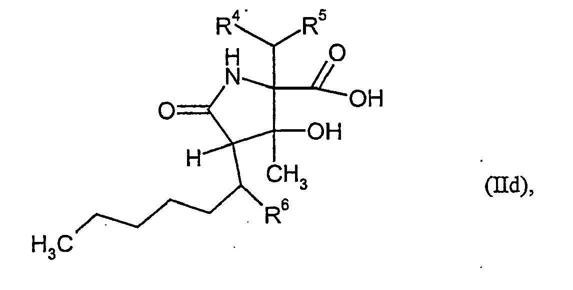 wherein
   R⁴, R⁵ and R⁶ have the meaning described above,
with thioles.

Formula (I) contains the compounds of formula (Ib), (Ic), (Id) and (Ie).

Formula (II) contains the compounds of formula (IIb), (IIc) and (IId).

Process [A] and [E] can be carried out as described in the experimental section or *Streptomyces* sp. JS 360 is fermented in an aqueous nutrient medium under submerged aerobic conditions. Typically the microorganism is fermented in a nutrient medium containing a carbon source and a proteinaceous material. Preferred carbon sources include glucose, brown sugar, sucrose, glycerol, starch, corn starch, lactose, dextrin, molasses, and the like. Preferred nitrogen sources include cottonseed flour, corn steep liquor, yeast, autolysed brewer's yeast with milk solids, soybean meal, cottonseed meal, corn meal, milk solids, pancreatic digest of casein, distillers' solids, animal peptone liquors, meat and bone scraps, and the like. Combinations of these carbon and nitrogen sources can be used advantageously. Trace metals, for example. zinc, magnesium, manganese, cobalt, iron and the like need not be added to the fermentation medium since tap water and unpurified ingredients are used as medium components.

Production of compounds can be induced at any temperature conductive to satisfactory growth of the microorganisms between about 23° and 32°C and preferably at about 28°C. Ordinarily, optimum production of compounds is obtained in about 2 to 6 days of fermentation, and preferably in about 4 to 5 days of fermentation. The fermentation broth normally remains weakly to moderately acidic during the fermentation, and advantageously the fermentation is terminated at pH of 4-4.5. The final pH is dependent, in part, on the buffers present, if any, and in part, on the initial pH of the culture medium. It is advantageously adjusted to about pH 6.5-7.5, and preferably 7.2, prior to sterilisation.

Production takes out in shake flask but also in solid media and stirred fermentors. When growth is carried out in shake flasks or large vessels and tanks, it is preferable to use the vegetative form, rather than the spore form, of the microorganism for inoculation to avoid a pronounced lag in the production of the compounds and the attendant inefficient utilisation of the equipment. Accordingly, it is desirable to produce a vegetative inoculum in an aqueous nutrient medium by inoculating this medium with an aliquot from a soil or a slant culture. When a young, active vegetative inoculum has thus been secured, it is transferred aseptically to other shake flasks or other suitable devices for fermentation of microorganisms. The medium in which the vegetative inoculum is produced can be the same as, or different from, that utilised for the production of compounds, as long as it is such that adequate growth of the microorganism is obtained.

In general, seeding of Streptomyces sp. JS360 and fermentation and the production of compounds in submerged aerobic fermentation in stirred vessels is utilised. The production is independent of used containers, fermentors and starter proceedings. The compounds can also be obtained by shake-flask culture. For large volume fermentations it is preferable to use a vegetative inoculum. The vegetative inoculum is prepared by inoculating small volume of culture medium with the spore form, mycelial fragments, or a lyophilised pellet of the organism. The vegetative inoculum is then transferred to a fermentation vessel where, after a suitable incubation time, compounds are produced in optimal yield.

As is customary in aerobic submerged culture process, sterile air is dispersed through the culture medium. For efficient growth of the organism, the volume of the air used is in the range of from about 0.25 to about 0.5 volume of air per volume of culture medium per minute (vvm). An optimum rate in a 10 1 vessel is about 0.3 vvm with agitation provided by conventional impellers rotating at about 240 rpm. Adding of small amount (i. e. 1 ml/l) of an antifoaming agent such as silicone to fermentations media is necessary if foaming becomes a problem. Preferred fermentation conditions and media are given in General Experimental Procedures

Compounds are present in the biomass of the fermentated Streptomyces sp. JS 360, as well as in the culture filtrate of the fermentation broth. The culture broth can be separated by filtering on a filter press.

A variety of procedures can be employed to isolate and purify the compounds from the fermentation broth, for example, by chromatographic adsorption procedures followed by elution with a suitable solvent, column chromatography, partition chromatography, and crystallisation from solvents and combinations thereof.

In the preferred recovery process, the compounds are extracted from the whole beer, from the mycelia or from extracts of the supernatant. The latter can be prepared by using adsorbant resins such as XAD, HP 20 or Bayer Lewapol. Column chromatography techniques, preferably over silica gels or modified silica gels, are used to perform the initial purification. Final purification of the compounds is preferably achieved by preparative High Performance Liquid Chromatography (HPLC).

The hydrogenation in process [B] can be carried out in the presence of an catalyst such as palladium/charcoal and hydrogen in a suitable solvent in a temperature range from 0°C to +100°C, at normal pressure or at elevated pressure up to 3 bar.

Suitable solvents are i.e. ethers such as diethyl ether, methyl-t-butyl ether, dioxan or tetrahydrofuran, alcohols such as methanol, ethanol, n-propanol, iso-propanol, n-butanol or t-butanol, preferred is methanol, ethanol, iso-propanol or tetrahydrofuran.

The hydration in process [C] can be carried out by hydroboration with oxidative work-up using e.g. diborane (B₂H₆) in tetrahydrofuran followed by hydrogen peroxide. Alternatively an epoxide can be generated and opened by reduction methods. All processes can be carried out in a suitable solvent in a temperature range from -78°C to +25°C, at normal pressure or at elevated pressure up to 3 bar.

Suitable solvents are i.e. tetrahydrofuran, diethyl ether, tert-butyl-methyl ether, and related solvents.

The oxidation in process [D] can be carried out by chiral or achiral dihydroxylation methods using potassium permanganate (KMnO₄) or osmium tetroxide (OsO₄). In the case of osmium tetroxide, catalytical amounts may be sufficient, when tert. amine N-Oxides e.g. N-Methyl-morpholine-N-oxide or other oxidants like potassium ferricyanide (K₃FeCN₆) are used. All processes can be carried out in a suitable solvent in a temperature range from 0°C to +100°C, at normal pressure or at elevated pressure up to 3 bar.

Suitable solvents are alcohols such as ethanol or t-butanol, with appropriate amounts of water added.

The reaction with thiols in process [F] can be carried out in the presence of a base such as triethylamine or diisopropylethylamine in a suitable solvent in a temperature range from 0°C to 50°C, at normal pressure.

Suitable solvents are i.e. tetrahydrofuran, dichloromethane, dimethylformamide, and related solvents.

The compounds according to the invention exhibit an unforeseeable, useful pharmacological and pharmacokinetic activity spectrum. They are therefore suitable for use as medicaments for the treatment and/or prophylaxis of disorders in humans and animals.

The compounds according to the invention are because of their pharmacological properties useful alone or in combination with other active components to provide an effective treatment of acute and chronic inflammatory processes such as toxic shock syndrome, endotoxic shock, tuberculosis, allergy, atherosclerosis, psoriatic arthritis, Reiter's syndrome, gout, traumatic arthritis, rubella arthritis and acute synovitis, rheumatoid arthritis, rheumatoid spondylitis, osteoarthritis, gouty arthritis and other arthritic conditions, sepsis, septic shock, gram negative sepsis, cerebral malaria, meningitis, ischemic and hemorrhagic stroke, neurotrauma / open or closed head injury, silicosis, pulmonary sarcososis, bone resorption disease, osteoporosis, restenosis, cardiac, brain and renal reperfusion injury, thrombosis, glomerulamephritis, chronic renal failure, diabetes, diabetic retinopathy, macular degeneration, graft vs. host reaction, allograft rejection, inflammatory bowel disease, Crohn's disease, ulcerative colitis, neurodegenerative disease, muscle degeneration, angiogenic disease, eczema, contact dermatitis, psoriasis, sunburn, conjunctivitis, adult respiratory distress syndrome (ARDS), chronic obstructive pulmonary disease (COPD), airway inflammation, asthma, fever, periodontal diseases, pyresis, Alzheimer's and Parkinson's diseases and pain, especially of COPD and asthma.

The compounds of the present invention are also useful for treatment of cancer such as ovarian cancer or colon cancer, tumor growth and metastasis, autoimmune disorders, cardiovascular diseases such as myocarditis, restenosis following angioplasty, renal diseases such as lupus, polycystic kidney disease, fungal infections, virus infection such as HIV, bacterial infection, dermatological diseases such as psoriasis, abnormal wound healing, keloids, immunological diseases such as autoimmunity, acute and delayed hypersensitivity, graft versus host disease, transplant rejection and neuroimmunological diseases such as multiple sclerosis and acute disseminated encephalomyelitis.

In another embodiment, the present invention relates to the composition containing at least one compound of general formula (I) and a pharmacologically acceptable diluent and the use of such composition for the treatment of acute and chronic inflammatory processes or cancer as well as the process for the preparation of such compositions, characterized in that the compounds of general formula (I) together with customary auxiliaries in brought into a suitable application form. The compounds of general formula (I) are therefor useful for the preparation of medicaments, especially of medicaments for the treatment of acute and chronic inflammatory processes, especially COPD, or cancer.

For the treatment of the above-mentioned diseases, the compounds according to the invention can exhibit non-systemic or systemic activity, wherein the latter is preferred. To obtain systemic activity the active compounds can be administered, among other things, orally or parenterally, wherein oral administration is preferred. To obtain non-systemic activity the active compounds can be administered, among other things, topically.

For parenteral administration, forms of administration to the mucous membranes (i.e. buccal, lingual, sublingual, rectal, nasal, pulmonary, conjunctival or intravaginal) or into the interior of the body are particularly suitable. Administration can be carried out by avoiding absorption (i.e. intracardiac, intra-arterial, intravenous, intraspinal or intralumbar administration) or by including absorption (i.e. intracutaneous, subcutaneous, percutaneous, intramuscular or intraperitoneal administration).

For the above purpose the active compounds can be administered per se or in administration forms.

Suitable administration forms for oral administration are, inter alia, normal and enteric-coated tablets, capsules, coated tablets, pills, granules, pellets, powders, solid and liquid aerosols, syrups, emulsions, suspensions and solutions. Suitable administration forms for parenteral administration are injection and infusion solutions.

The active compound can be present in the administration forms in concentrations of from 0.001 - 100 % by weight; preferably the concentration of the active compound should be 0.5 - 90% by weight, i.e. quantities which are sufficient to allow the specified range of dosage.

The active compounds can be converted in the known manner into the above-mentioned administration forms using inert non-toxic pharmaceutically suitable auxiliaries, such as for example excipients, solvents, vehicles, emulsifiers and/or dispersants.

The following auxiliaries can be mentioned as examples: water, solid excipients such as ground natural or synthetic minerals (e.g. talcum or silicates), sugar (e.g. lactose), non-toxic organic solvents such as paraffins, vegetable oils (e.g. sesame oil), alcohols (e.g. ethanol, glycerol), glycols (e.g. polyethylene glycol), emulsifying agents, dispersants (e.g. polyvinylpyrrolidone) and lubricants (e.g. magnesium sulphate).

In the case of oral administration tablets can of course also contain additives such as sodium citrate as well as additives such as starch, gelatin and the like. Flavour enhancers or colorants can also be added to aqueous preparations for oral administration.

For the obtainment of effective results in the case of parenteral administration it has generally proven advantageous to administer quantities of about 0.001 to 100 mg/kg, preferably about 0.01 to 1mg/kg of body weight. In the case of oral administration the quantity is about 0.01 to 100 mg/kg, preferably about 0.1 to 10 mg/kg of body weight.

In spite of this, it can be necessary in certain circumstances to depart from the amounts mentioned, namely as a function of body weight, application route, individual behaviour towards the active component, manner of preparation and time or interval at which application takes place. It can for instance be sufficient in some cases to use less than the aforementioned minimum amount, while in other cases the upper limit mentioned will have to be exceeded. In the case of the application of larger amounts, it can be advisable to divide them into a plurality of individual doses spread through the day.

The percentages in the tests and examples which follows are, unless otherwise stated, by weight; parts are by weight. Solvent ratios, dilution ratios and concentrations reported for liquid/liquid solutions are each based on the volume.

### A. Examples

### The following abbreviations are used in the descriptions

- ACN: acetonitrile
- aq.: aqueous
- Bn: benzyl
- BOP: benzotriazole-1-yloxytris(dimethylamino)-phosphoniumhexafluorophosphate
- DCI: direct chemical ionisation
- DCM: dichloromethane
- DMF: N,N-dimethylformamide
- DMSO: dimethylsulfoxide
- EDTA: ethylenediamine tetra-acetic acid
- ESI: electro-spray ionisation
- FCS: Fetal calf serum
- h: hour / hours
- HPLC high: pressure liquid chromatography
- LC/MS: liquid chromatography-coupled mass spectroscopy
- min.: minute(s)
- mp: melting point
- MS: mass spectroscopy
- NMR: nuclear magnetic resonance spectroscopy
- PBS: Phosphate buffered saline
- RP: reverse phase (HPLC)
- Rₜ: retention time (HPLC)
- rt: room temperature
- SDS: Sodium dodecyl sulphate
- TFA: trifluoroacetic acid
- THF: tetrahydrofuran
- UV: ultraviolet
- UV/Vis: ultraviolet-visual
- % of th.: % of theoretical yield

### General Experimental Procedures

Chemicals are obtained in analytical grade from Merck (Darmstadt, Germany) or Sigma-Aldrich (Deisenhofen, Germany). NMR spectra are recorded in DMSO-d₆ using a Bruker DRX 500 spectrometer (operating at 500.13 MHz proton frequency).

HPLC-MS analyses are performed using a Agilent HP1100 liquid chromatograph coupled with a LCT mass spectrometer (Micromass, Manchester, UK) in the positive and negative electrospray ionisation (ESI) mode, based on slight modification of a previously described method (M. Stadler et al., Phytochemistry 2001, 56, 787-793). A Waters symmetry column is used as stationary phase. Mobile phase A: 0.1 % formic acid in water, mobile phase B: 0.1 % formic acid in acetonitrile; gradient: 0-1 min. 100 % A, from 1-6 min. to 90 % B, from 6 to 8 min to 100 %B, from 8-10 min 100 % B. LC-MS spectra are recorded in the range of molecular weights between 150 and 1.600.

HPLC-UV/Vis analyses are carried out in analogy to M. Stadler et al., Mycol. Res., 2001, 105, 1190-1205 on a HP 1100 Series analytical HPLC system (Agilent, Waldbronn, Germany) comprising a G 1312A binary pump system, a G 1315A diode array detector, a G 1316A column compartment, a G 1322A degaser and a G 1313A autoinjector. As mobile phase, 0.01% phosphoric acid: acetonitrile is chosen, while a Merck (Darmstadt, Germany) Lichrospher RP 18 column (125 x 4 mm, particle size 7 µm) serves as stationary phase. Aliquots of the samples (representing 2 - 10 µg of methanol-soluble materials, according to the concentrations of main metabolites) are analysed at 40°C with a flow of 1 ml/min in the following gradient: Linear from 0% acetonitrile to 100% acetonitrile in 10 min, thereafter isocratic conditions at 100% acetonitrile for 5 min; followed by regeneration of the column for 5 min. HPLC-UV chromatograms are recorded at 210 nm with a reference wavelength of 550 nm and a bandwidth of 80 nm. Diode array detection (DAD) is employed to record HPLC-UV/Vis spectra in the range of 210 - 600 nm. The HP ChemStation software allows for an automated search for calibrated standard compounds in crude extracts.

Preparative HPLC is performed at room temperature on a preparative HPLC system (Gilson Abimed, Ratingen, Germany), comprising Gilson Unipoint software, 306 binary pump system, 205 fraction collector, 119 UV-Vis detector, 806 manometric module, and 811C dynamic mixer, using different gradients and stationary phases as described below.

NMR spectra are recorded on a Bruker DMX500, operating at 500.13 MHz proton frequency. All spectra are measured in DMSO-d₆ solution at 302 K. The solvent peak is used as internal reference for both proton and carbon chemical shifts (δ_{H}: 2.50, δ_{C}: 39.5).

### Characterisation and maintenance of strain Streptomyces sp. JS360

### Culture media

Yeast-Malt-Glucose (YMG) medium: D-glucose 0.4%, malt extract 1%, yeast extract 0.4%, pH 7.2.

Q6 medium: D-glucose 0.4%, glycerol 2%, cotton seed meal 1%, tap water, pH 7.2.

C medium: D-glucose 1%, yeast extract 1%, NZ amine (Sheffield Chemicals, Sheffield, U,.K., Lot ONA 20 2) 0.5%, soluble starch 2%, no pH adjustment.

GS medium: D-glucose 2%, deoiled soymeal (Soyamin 50 T, Degussa, Düsseldorf, Germany) 2%, soluble starch 2%, calcium carbonate 0.5%, sodium chloride 0.25%, magnesium sulfate 0.05%, potassium dihydrogen phosphate 0.025%, pH adjustment to 6.5-6.8.

MC medium: D-glucose 1%, yeast extract 0.5%, deoiled soymeal (Soyamin 50 T, Degussa, Düsseldorf, Germany) 1%, soluble starch 1%, sodium chloride 0.5%, calcium carbonate 0.3%, pH adjustment to 7.2 (0.1N sodium hydroxide solution).

MCPM medium: Diamalt Maltzin hell (Meistermarken GmbH, Bremen, Germany) 4.5%, NZ amine (Sheffield Chemicals, Sheffield, U.K., Lot ONA 20 2) 1%, sodium chloride 0.3%, potassium dihydrogen phosphate 0.1%, magnesium sulfate 0.05%, ferrous sulfate 0.01%, pH 6.8.

MS medium: Mannitol 2%, Soymeal defatted (Soyamin 50 T, Degussa, Düsseldorf, Germany) 2%, calcium carbonate 0.3%, pH adjusted to 7.5.

SP medium: Mannitol 3%, yeast extract 0.75%, soluble starch 0.2%, soy peptone (Merck, Darmstadt, Germany # 107212.0500)) 0.5%, pH adjustment to 6.0 (hydrochloric acid).

Strain JS360 is obtained from a soil sample collected in Japan. It is maintained at the Bayer AG culture collection (Wuppertal, Germany) in 10% glycerol under liquid nitrogen. It has also been deposited at DSMZ (Deutsche Sammlung für Mikroorganismen und Zellkulturen, Mascheroder Weg 1b, D-38124 Braunschweig, Germany), on November 27, 2002 under the designation number DSM 15324.

### Strain identification

The morphological, cultural and physiological characteristics of strain JS360 indicate that the strain constitutes an undescribed species of the genus *Streptomyces.*

### Morphology

On YMG medium, single colonies of strain JS360 attain a diameter of 24 mm after incubation for 12 days at 28°C. The colonies develop a white, fluffy aerial mycelium, while the substrate mycelium is creamish. The reverse of the culture is reddish brown, and a reddish pigment is released into the medium.

### 16S rDNA (SEQ ID NO: 1) sequencing

Comparisons of the 16S rDNA (SEQ ID NO: 1) sequences are carried out to further characterize strain JS360 on its taxonomic position. Thus, DNA extraction and sequencing of the main part of the 16S rRNA gene is amplified in analogy to Mincer TJ, Jensen PR, Kaufmann CA, Fenical W. 2002. Appl. Environ. Microbiology 60 (10) 5005-5011 a PCR using primers 41f and 1486r-P (unpublished), applying a standard thermal profile with an annealing temperature of 50°C. Amplification products are purified using DNA binding paramagnetic beads (Mag Prep PCR Clean Up Kit, Tecan Schweiz AG, Hornbrechtikon, Switzerland) using the protocol supplied by the manufacturer.

Nucleotide sequences are obtained by cycle sequencing using the Thermo Sequenase Cy5.5 Dye Terminator Cycle Sequencing Kit (Amersham Biosciences), primer 41f, and the LI-COR 4200 Genetic Analyzer (LI-COR Inc. Lincoln, Nebraska, USA). A search for sequences similar to the ones determined and accompanying alignments with the best matches are obtained by FASTA as provided as an on-line service by the Europaean Bioinformatics Institute (EBI) (http://www.ebi.ac.uk/fasta33/). The sequences showing the best matches are obtained from the database to be used as input for the MegAlign module of the LASERGENE software (DNASTAR Inc, Madison, Wisconsin, USA.). Sequences of *Salinospora* sp. published by Mincer et al., Appl. Environm. Microbiol., 2002, 68, 5005-5011, are also taken into consideration.

The following nucleotide sequences are used for comparison:

| **Sequence code** | **Origin** |
|---|---|
| AY040623 | *Salinospora* sp. CNH964 16S ribosomal RNA gene, partial sequence. |
| AY040618 | *Salinospora* sp. CNH536 16S ribosomal RNA gene, partial sequence. |
| AY040619 | *Salinospora* sp. CNH643 16S ribosomal RNA gene, partial sequence. |
| AY040620 | *Salinospora* sp. CNH646 16S ribosomal RNA gene, partial sequence. |
| AY040621 | *Salinospora* sp. CNH725 16S ribosomal RNA gene, partial sequence. |
| AY040617 | *Salinospora* sp. CNH440 16S ribosomal RNA gene, partial sequence. |
| AY039455 | Earthworm burrow bacterium B38M1 16S ribosomal RNA gene, partial sequence. |
| AY039483 | Soil bacterium S31M1 16S ribosomal RNA gene, partial sequence. |
| AJ399487 | *Streptomyces cinnabarinus* partial 16S rRNA gene |

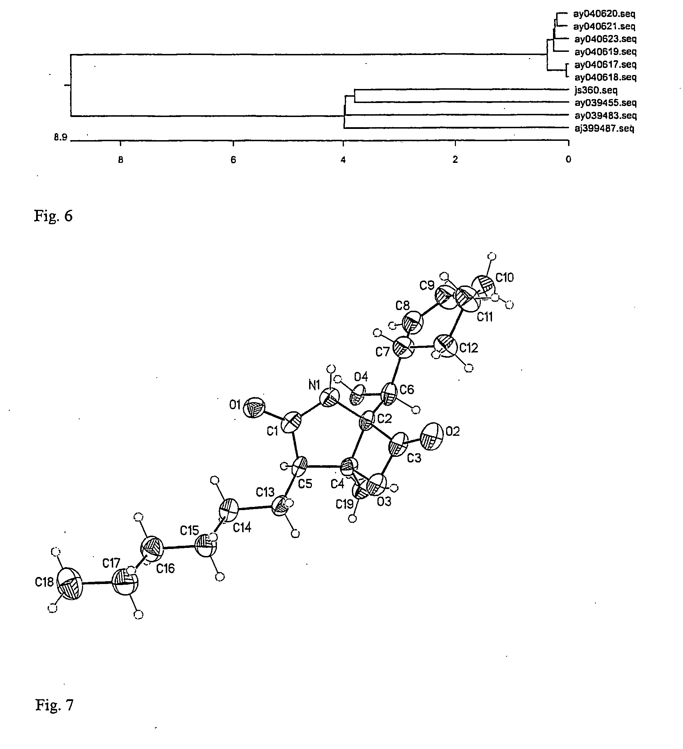

About 240 bases of sequence are obtained from strain JS360 (Fig. 5). The sequence is used as a FASTA input to search for similar sequences in the EMBL databank. The sequence is found to be closely related to 16S rRNA sequences of members of *Streptomyces.* Among the three best matches are two unnamed streptomycete isolates from soil and an earthworm, respectively, and one isolate of *Streptomyces cinnabarinus.* The similarity between these three and the sequence obtained from JS360 is in the range of 91%. No identical sequence is found in the database. An alignment (Clustal method) is done including the sequence of JS360 and three of the most similar sequences as well as six sequences of *Salinospora* species. From the alignment, a dendrogram is constructed to demonstrate the phylogenetic relationships. A clear destinction is found between *Salinospora* sp. and the group including JS360 (Fig. 6). The results reveal JS360 to be clearly distinct from *Salinospora* spp. The strain belongs to the genus *Streptomyces* as concluded from the alignment of its partial 16S rDNA sequence.

### Fermentation and extraction of strain JS360

### 1: Seed culture

Two ml of a 10% glycerol culture are used to inoculate 11 Erlenmeyer flasks containing 150 ml of sterile YMG medium and propagated on a rotary shaker at 28°C and 240 rpm for 72-96 h.

### 2: Fermentation of strain JS360 in flask scale

After inoculation from a well-grown YMG seed culture (2 ml inoculum per flask), strain JS360 is propagated in ten 11 Erlenmeyer flasks containing 150 ml of Q6 medium (see above) and propagated on a rotary shaker at 28°C and 240 rpm for 118 h. During fermentation, daily samples are taken. The pH is determined, and free glucose is estimated using Bayer Diastix Harnzuckerstreifen. The wet mycelium is separated from the fluid by centrifugation (10 min. at 3000x g) and extracted with 2 1 of acetone. The acetone is evaporated in vacuo (40°C). The remaining aqueous residue is diluted with water to 500 ml and extracted three times with equal amounts of ethyl acetate. The combined organic phases are dried over sodium sulfate and evaporated in vacuo (40°C) to yield 830 mg of crude extract, which is thereafter subjected to preparative HPLC as described below (isolation).

The culture fluid is applied onto an adsorption column containing 500 ml of Bayer Lewapol CA 9225 resin and rinsed with 11 water. The column is eluted with 1.5 1 acetone:methanol.4:1. The solvent is evaporated in vacuo (40°C). The remaining aqueous residue is diluted with water to 500 ml and extracted three times with equal amounts of ethyl acetate. The combined organic phases are dried over sodium sulfate and evaporated in vacuo (40°C) to yield 650 mg of crude extract, which is thereafter subjected to preparative HPLC as described below (isolation).

### 3: Fermentation of strain JS360 in 301 scale (stirring fermentor)

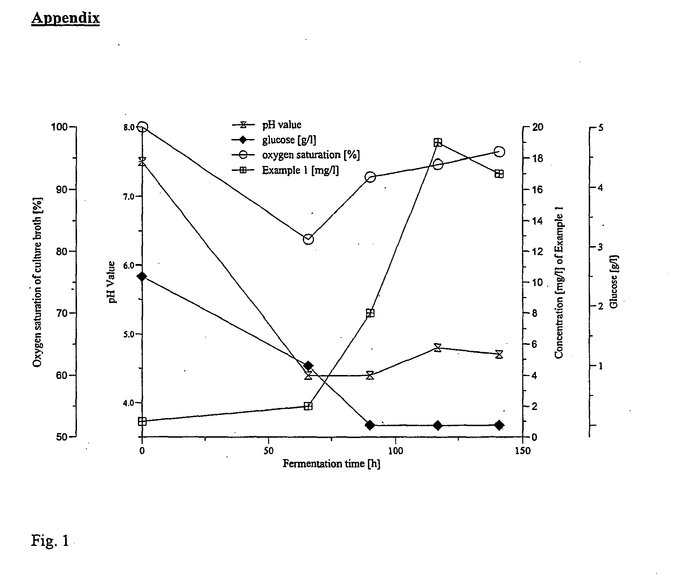

A 40 l Biostat P fermentor (Braun Bioengeneering, Melsungen, Germany) containing 30 1 of Q6 medium is sterilized in situ (1 h at 121°C and 1.1 bar) and inoculated with two well-grown 150 ml YMG seed cultures that have been propagated for 76 h. The production culture is grown under stirring (240 rpm) and aeration (0.3 vvm). The pH is determined, and free glucose is estimated using Bayer Diastix Harnzuckerstreifen. In addition, the fermentor is equipped with a Braun oxygen electrode to determine oxygen saturation of the culture broth. Analytical HPLC of crude extracts prepared from 50 ml samples taken under sterile conditions and extracted with equal amounts of ethyl acetate serve as a means of detection for example 1. Examples 2 to 5 and 7 are also detected during fermentation by HPLC-MS but cannot be estimated in the native crude extracts, due to limited amounts and co-eluting other metabolites with similar retention times in the employed HPLC system. The ethyl acetate extracts are dried over sodium sulfate, evaporated to dryness, redissolved in methanol and analyzed using the HPLC-UV systems described in General Experimental Procedures. A typical time course of the fermentation of JS360 in 30 1 Q6 medium is depicted in Fig. 1. While the culture is fully saturated as deduced from the oxygen saturation values, the pH drops to values of ca. 4.5. After the free glucose in the medium is consumed, production of example 1 as estimated by analytical HPLC methodology starts at about 60 h of fermentation and reaches an optimum after 114 h. Then, the culture is harvested because at later stages degradation of example 1 is observed. After harvest of the culture, the fluid is separated from the mycelium by centrifugation (10 min. at 3000 x g) and applied onto a column filled with Bayer Lewapol CA 9225 adsorption resin and rinsed with 5 1 water. The column is thereafter eluted with 6 1 acetone:methanol 4:1. The eluates are evaporated *in vacuo* (40°C) to yield an aqueous residue, which is diluted to 11 with water and extracted three times with 11 ethyl acetate. The organic phases are combined, dried over sodium sulfate and evaporated *in vacuo* (40°C). The resulting extract (22.7 g) is thereafter subjected to preparative HPLC as described below (isolation).

The mycelium is extracted three times with each 5 1 of acetone, and the acetone is evaporated *in vacuo* (40°C) to yield an aqueous residue, which is diluted to 11 with water and extracted three times with 11 ethyl acetate. The organic phases are combined, dried over sodium sulfate and evaporated *in vacuo* (40°C). The resulting extract (13.4 g) is thereafter subjected to preparative HPLC as described below (isolation).

### 4. Fermentation in other culture media (flask scale)

Strain JS 360 is propagated in various other culture media in attempts to optimize production of example 1 and chemically related metabolites. For this purpose, shake flask fermentations are carried out in a similar manner as described for the one in Q6 medium (see 2. above). 11 Erlenmeyer flasks containing 150 ml of the media are thus propagated on a rotary shaker at 28°C and 240 rpm for up to 118 h. During fermentation, daily samples are taken. The pH is determined, and free glucose is estimated using Bayer Diastix Harnzuckerstreifen. Aliquots of the culture broth (50 ml) are extracted with ethyl acetate. These ethyl acetate extracts are dried over sodium sulfate, evaporated to dryness, redissolved in methanol and analyzed using the HPLC-UV and HPLC-MS systems described in General Experimental Procedures. By comparison of retention times and spectra, example 1 and related compounds are detected in the following culture media: YM medium, C medium, GS medium, MC medium, MCPM medium, MS medium, and SP medium after 72-96 hours of fermentation. The highest yields of example 1, however, are observed in Q6 and GS media.

### Example 1

### (1R,4R,5S)-1-[(S)-(1S)-2-cyclohexen-1-yl(hydroxy)methyl]-4-hexyl-5-methyl-6-oxa-2-azabicyclo[3.2.0]heptane-3,7-dione

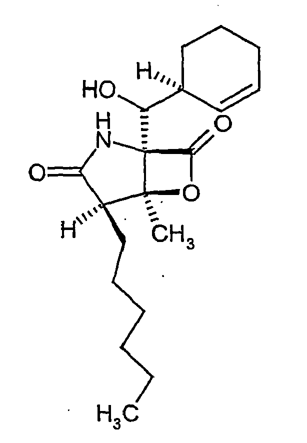

Preparation see below.

### Example 2

### (1R,4R,5S)-1-[(S)-(1S)-2-cyclohexen-1-yl(hydroxy)methyl]-4-[1-hydroxy-hexyl]-5-methyl-6-oxa-2-azabicyclo[3.2.0]heptane-3,7-dione

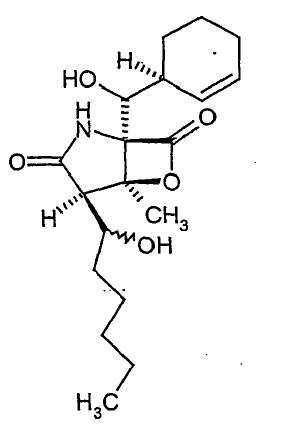

Preparation see below.

### Example 3

### (1R,4R,5S)-1-[(1R)-2-cyclohexen-1-ylmethyl]-4-hexyl-5-methyl-6-oxa-2-aza-bicyclo[3.2.0]-heptane-3,7-dione

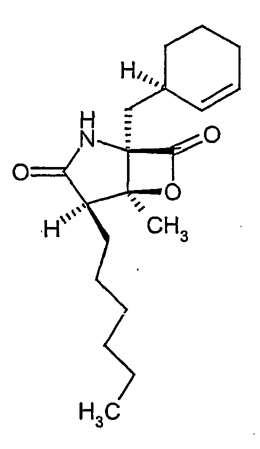

Preparation see below.

### Example 4

### (3S,4R)-2-[(S)-(1S)-2-cyclohexen-1-yl(hydroxy)methyl]-3-hydroxy-4-[1-hydroxy-hexyl]-3-methyl-5-oxo-D-proline

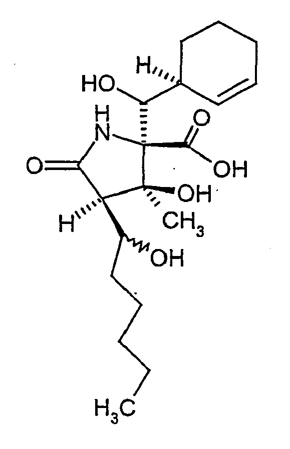

Preparation see below.

### Example 5

### N-acetyl-S-({(2R,3S,4R)-2-[(S)-(1S)-2-cyclohexen-1-yl(hydroxy)methyl]-4-hexyl-3-hydroxy-3-methyl-5-oxo-2-pyirolidmyl}carbonyl)cysteine

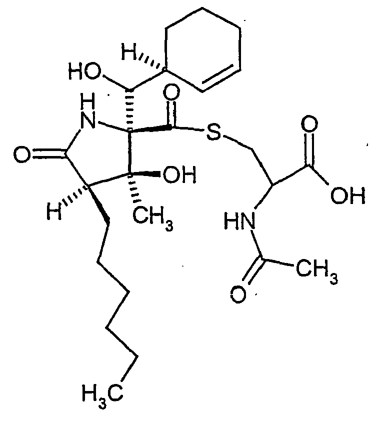

Preparation see below.

### Example 6

### Methyl-N-acetyl-S-({(2R,3S,4R)-2-[(S)-(1S)-2-cyclohexen-1-yl(hydroxy)methyl]-4-hexyl-3-hydroxy-3-methyl-5-oxo-2-pyrrolidinyl}carbonyl)cysteinate

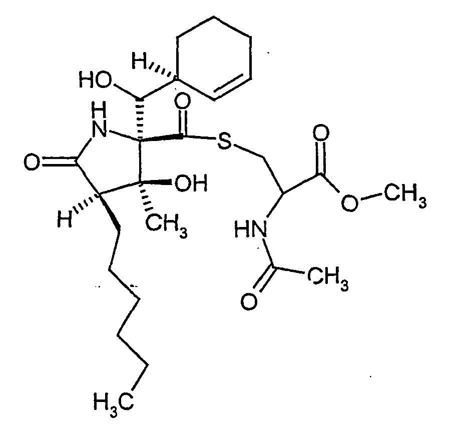

Preparation see below.

### Example 7

### (3S,4R)-2-[(S)-(1S)-2-cyclohexen-1-yl(hydroxy)methyl]-3-hydroxy-4-hexyl-3-methyl-5-oxo-D-proline

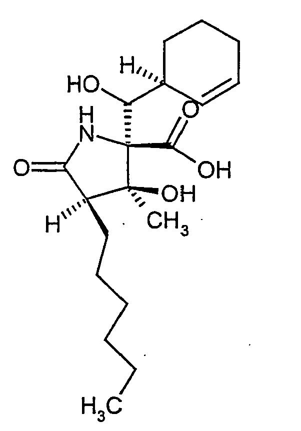

Preparation see below.

The stereochemistry of examples 2 to 7 is drawn in analogy to the structure of example 1 which is determined via X-ray analysis.

### Isolation of examples 1 to 7

### 1. Materials from shake flask fermentations

The crude extracts (620 mg from the mycelium and 830 mg from the culture fluid, respectively) are dissolved in 5 ml of methanol, filtered through a Bond Elut C18 500 mg solid phase extraction cartridge (Baker, Deventer, The Netherlands) and applied onto a MZ Analysentechnik (Mainz, Germany) Kromasil RP 18 column (particle size, 7 µm; 250 x 40 mm). As mobile phase, a gradient of 0.01% TFA: acetonitrile is employed at a flow of 10 ml/min:20 % acetonitrile at t = 0 min; linear gradient: 20% to 50% acetonitrile in 40 min; thereafter linear gradient from 50% to 100% acetonitrile in 20 min; thereafter isocratic conditions at 75% acetonitrile for 30 min, thereafter regeneration of the column. Fractions are combined according to UV adsorption at 210 nm. Example 1 is eluted at a retention time (Rₜ) of 80-83 min. and is obtained in amounts of 14 mg from the mycelial extract and 1.5 mg from the culture fluid extract, respectively. Examples 2 to 5 and 7 are located in minor intermediate fractions and not isolated to purity from this extract, while example 6 is not detected at all.

### 2: Materials from 301 scale fermentation

Aliquots of 2.5-3 grams of the crude extracts which are prepared as described above (13.4 g from mycelium, 22.7 g from culture fluid) are dissolved in 5 ml of methanol, filtered through a Bond Elut C18 500 mg solid phase extraction cartridge (Baker, Deventer, The Netherlands) and applied onto a MZ Analysentechnik (Mainz, Germany) Kromasil RP 18 column (particle size, 7 µm; 250 x 40 mm; mobile phase, 0.01% TFA: acetonitrile). As mobile phase, a gradient of 0.01% TFA: acetonitrile is employed at a flow of 10 ml/min: 20 % acetonitrile at t = 0 min; linear gradient: 20% to 50% acetonitrile in 40 min; thereafter linear gradient from 50 % to 100% acetonitrile in 20 min; thereafter isocratic conditions at 75% acetonitrile for 30 min, thereafter regeneration of the column. Fractions are combined according to UV adsorption at 210 nm. Five bioactive intermediate products are thus obtained. Their retention times (Rₜ) in this gradient system are observed as follows: 61-64 min. for intermediate product 1 (containing example 4 and 7), 65-71 min. for intermediate product 2 (containing examples 5 and 6), 72-79 min. for intermediate product 3 (containing example 2), 79-85 min. for intermediate product 4 (containing example 1) and 86-93 min. for intermediate product 5 (containing example 3).

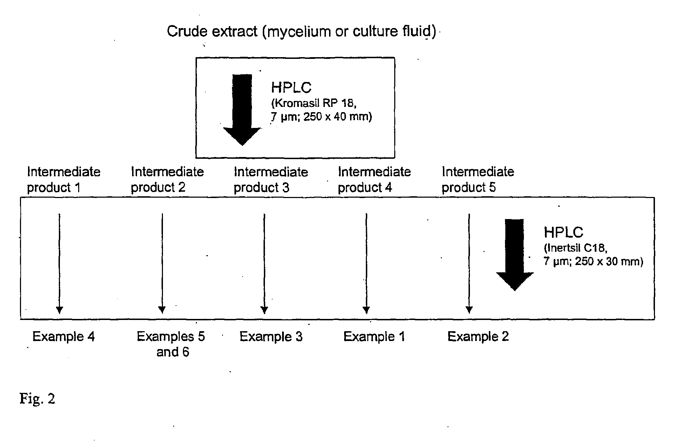

Final purification of active components in intermediate products 1 to 5 is obtained by preparative reversed phase HPLC, using a flow of 7 ml/min and a MZ Analysentechnik Inertsil C18 column (7 µm; 250 x 30 mm) as stationary phase and the following gradient. Isocratic conditions from t = 0 min => t = 30 min; thereafter linear gradient from 30% acetonitrile => 100% acetonitrile in 50 min, thereafter isocratic conditions (100% acetonitrile) for 20 min, thereafter regeneration of the column. Yields and Rₜ of examples 1 to 6 are summarized in table 1. A general scheme for isolation is illustrated in Fig. 2.

**Table 1**

| **Example** | **Yield** **(Culture fluid extract)** | **Yield** **(Mycelial ectract)** | **Rₜ (min)** |
|---|---|---|---|
| 1 | 14 mg | 160 mg | 59-65 |
| 2 | 29 mg | 43 mg | 33-35 |
| 3 | 5 mg | 18 mg | 77-80 |
| 4 | 12 mg | 17 mg | 51-54 |
| 5 | 2 mg | 14 mg | 69-71 |
| 6 | (not isolated) | 2 mg | 72-73 |
| 7 | 19 mg | (not isolated) | 55-58 |

### Characterization of example 1 to 7

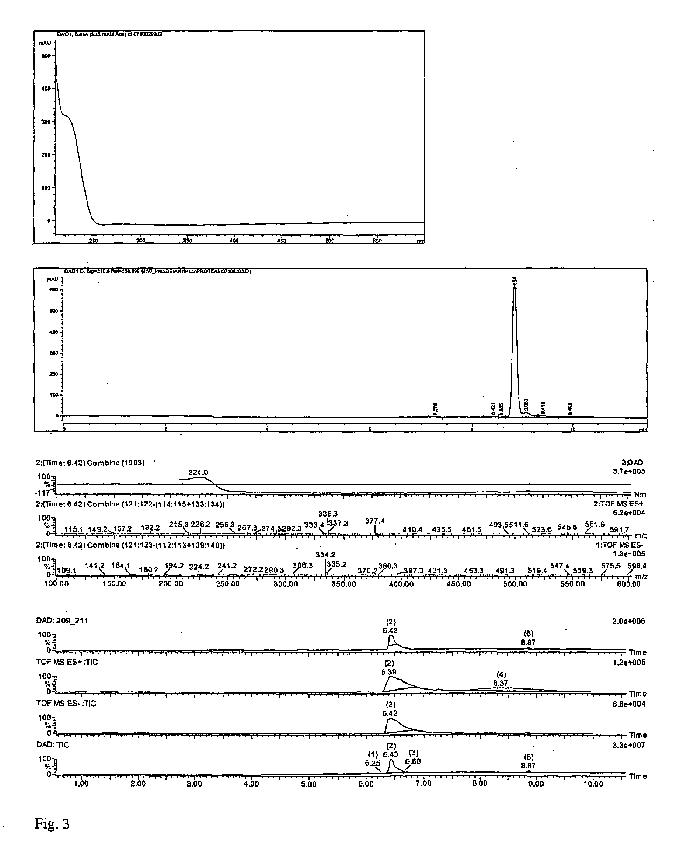

Example 1 to 6 are detected by HPLC-UV and HPLC-MS using the methods described in General Experimental Procedures. Their characteristics in analytical HPLC systems are summarized in table 2. The detection of example 1 in the employed gradients is illustrated in Fig. 3. While examples 1, 2, 4 and 7 give conclusive results regarding their molecular peaks, the LC-MS of example 3 only reveals the molecular peak in the positive ESI mode, while due to loss of carbon dioxide in the negative ESI mode, a smaller major mass fragment is observed. In examples 5 and 6, dimers are readily formed under the employed HPLC-MS conditions, and the major LC-MS signal thus relates to these dimers, while the molecular peaks only constitute minor signals. These characteristics also serve to identify the examples by analytical HPLC in fermentation broths and intermediate fractions obtained during extraction, downstream processing and chromatography.

**Table 2**

| **Example** | **Rₜ (HPLC-UV-Vis) [min]** | **Rₜ (HPLC-MS) [min]** | **Molecular peak m/z (pos. ESI)** | **Molecular peak m/z (neg. ESI)** |
|---|---|---|---|---|
| **1** | 8.95-8.97 | 6.39-6.43 | 336 (M+H)⁺ | 334 (M-H)⁻ |
| **2** | 7.75-7.85 | 5.73-5.81 | 352 (M+H)⁺ | 350 (M-H)⁻ |
| **3** | 9.74-9.76 | 6.86-6.90 | 320 (M+H)⁺ | 276 (M-CO₂-H)⁺ |
| **4** | 6.85-6.87 | 5.28-5.30 | 368 (M+H)⁺ | 370 (M-H)⁻ |
| **5** | 7.41-7.43 | 5.61-5.63 | 499 (M+H)⁺; 997 (2M+H)⁺ | 497 (M-H)⁻; 995 (2M-H)⁻ |
| **6** | 8.00-8.05 | 5.81-5.84 | 513 (M+H)⁺; 1025 (2M+H)⁺ | 511 (M-H)⁻ |
| **7** | 7.48-7.51 | 5.57-5.61 | 354 (M+H)⁺ | 352 (M-H)⁻ |

The structures of examples 1 to 7 are determined by low-resolution and high-resolution LC-MS spectrometry and by one- and two-dimensional NMR (nuclear magnetic resonance) spectroscopy. For instrumental parameters see General Experimental Procedures.

NMR data reveal the presence of a cis-double bond inside a cyclohexyl ring. The close analysis of HSQC, HMBC and COSY/TOCSY data allows to establish the bicyclic ring structure, which, together with the cyclohexenylcarbinol moiety, is identical to that found in *Salinosporamide A.* HSQC data point toward the presence of at least two methyl groups in each molecule. Together with TOCSY and HMBC, a non-branched hexyl moiety is identified. An unambiguous crosspeak in the COSY spectrum locates this chain at the 2-position in the heterocyclic ring system. Examples 7 (as compared to example 1) and example 4 (as compared to example 2), are revealed by NMR and MS data to constitute the respective seco-forms of the corresponding beta-lactone molecules. The presence of an additional hydroxyl group (compared to Salinosporamide A) at the 12-position (examples 2 and 4) becomes evident because of the multiplicity and the characteristic carbon and proton chemical shifts.

The NMR spectra of examples 5 and 6 show a complete new subset of signals that belong to an N-acylated cysteine moiety. The N-acetyl-cysteine is linked to the heterocylic ring structure via the carbonyl group of the former beta lactone ring, or the carboxyl group of example 7, respectively. The thioester link is identified by its carbonyl chemical shift ( > 200 ppm) and HMBC derived connectivity to the cysteine beta-hydrogens. All connectivities inside the cysteine residue are established by assigning the corresponding signals in HMBC and COSY spectra. Thus, the structures of examples 5 and 6 are analogous to that of lactacystin.

### Spectroscopic data

### Example 1

¹H-NMR (500 MHz, DMSO-d₆): δ = 0.87 (t), 1.28 (m), 1.29 (m), 1.23 (m), 1.40 (m), 1.45 (m), 1.47 (m), 1.54 (m), 1.58 (m), 1.68 (m), 1.74 (s), 1.80 (m), 1.90 (m), 2.29 (m), 2.41 (t), 3.65 (m), 5.47 (m), 5.73 (m), 5.81 (m), 8.92 (s).

¹³C-NMR (DMSO-d₆): δ = 13.8, 21.7, 21.8, 24.2, 25.2, 26.1, 26.8, 28.5, 30.8, 37.3, 47.5, 69.3, 78.4, 86.4, 127.8, 128.6, 169.1, 174.1.

### Example 2

¹H-NMR (500 MHz, DMSO-d₆): δ = 0.88 (t), 1.27 (m), 1.29 (m), 1.35 (s), 1.37 (m), 1.49 (m), 1.58 (m), 1.59 (m), 1.71 (m), 1.75 (m), 1.93 (m), 2.35 (d), 2.76 (m), 3.49 (m), 4.00 (d), 4.68 (m), 5.70 - (m), 5.91 (m), 6.11 (br.), 8.52 (s).

¹³C-NMR (DMSO-d₆): δ = 13.4, 21.0, 21.4, 23.7, 24.2, 29.6, 30.1, 31.5, 36.1, 54.6, 69.4, 75.0, 76.0, 76.5, 127.9, 170.9, 172.3.

### Example 3

¹H-NMR (500 MHz, DMSO-d₆): δ = 0.88 (t), 1.27 (m), 1.30 (m), 1.31 (m), 1.33 (m), 1.46 (m), 1.48 (m), 1.50 (m), 1.61 (s), 1.62 (m), 1.63 (m), 1.76 (m), 1.92 (m), 2.27 (m), 2.55 (t), 5.45 (m); 5.68 (m), 8.98 (s).

¹³C-NMR (DMSO-d₆): δ = 13.3, 19.6, 21.4, 24.0, 24.2, 26.5, 28.2, 28.5, 29.9, 30.9, 32.5, 46.7, 74.0, 86.1, 127.7, 130.9, 170.4, 170.8.

### Example 4

¹H-NMR (500 MHz, DMSO-d₆): δ = 0.82 (m), 1.17 (m), 1.20 (m), 1.24 (m), 1.32 (m), 1.47 (m), 1.60 (m),1.62 (m), 1.63 (m), 1.84 (m), 2.08 (m) 2.44 (d), 3.68 (m), 3.80 (m), 5.60 (m), 5.76 (m).

¹³C-NMR (DMSO-d₆): δ = 13.1, 20.1, 20.6, 21.0, 23.5, 23.6, 30.5, 33.5, 37.7, 52.7, 67.1, 73.6, 75.2, 80.1,126.3,128.6,171.2,176.5.

### Example 5

¹H-NMR (500 MHz, DMSO-d₆): δ = 0.87 (m), 1.09 (m), 1.24 (m), 1.25 (m), 1.27 (m), 1.33 (m), 1.35 (m), 1.37 (m), 1.44 (m), 1.46 (m), 1.50 (m), 1.61 (m), 1.64 (m), 1.84 (s), 1.87 (m), 2.13 (m), 2.47 (t), 2.96 (m), 3.30 (m), 3.78 (m), 4:36 (m), 5.64 (m), 5.79 (m).

¹³C-NMR (DMSO-d₆): δ = 13.9, 20.8, 21.7, 22.0, 22.1, 22.2, 23.4, 24.3, 26.8, 27.7, 28.7, 29.2, 31.1, 38.1, 50.3, 51.0, 75.3, 79.7, 80.6, 127.0, 129.3, 169.3, 178.9, 201.2.

### Example 6

¹H-NMR (500 MHz, DMSO-d₆): δ = 0.87 (m), 1.09 (m), 1.24 (m), 1.25 (m), 1.27 (m), 1.33 (m), 1.35 (m), 1.37 (m), 1.44 (m), 1.46 (m), 1.50 (m), 1.61 (m), 1.64 (m), 1.84 (s), 1.87 (m), 2.13 (m), 2.47 (t), 3.00 (m), 3.24 (m), 3.64 (s), 3.78 (m), 4.39 (m), 5.64 (m), 5.79 (m).

¹³C-NMR (DMSO-d₆): δ = 13.6, 20.8, 21.7, 22.0, 22.1, 23.4, 24.3, 26.8, 27.7, 28.7, 29.3, 31.1, 38.1, 50.3, 51.4, 51.8, 75.3, 79.7, 80.6, 127.0, 129.3, 169.3, 171.0, 178.9, 201.2.

### Example 7

¹H-NMR (500 MHz, DMSO-d₆): δ = 0.86 (t), 1.25 (m), 1.26 (m), 1.33 (m), 1.34 (m), 1.36 (m), 1.44 (m), 1.46 (s), 1.51 (m), 1.66 (m), 1.67 (m), 1.88 (m), 2.12 (m), 2.45 (t), 3.77 (d), 5.64 (m), 5.82 (m), 7.66 (s).

¹³C-NMR (DMSO-d₆): δ = 13.8, 20.3, 21.5, 21.7, 23.3, 24.4, 26.5, 27.9, 28.9, 31.0, 38.5, 50.5, 74.6, 75.5, 80.4, 127.4, 129.7, 177.5.

### Interpretation of the NMR-peak-lists:

example 1: R¹ = H, R² = OH, example 2: R¹ = OH, R² = OH, example 3: R¹ = H, R² = H. 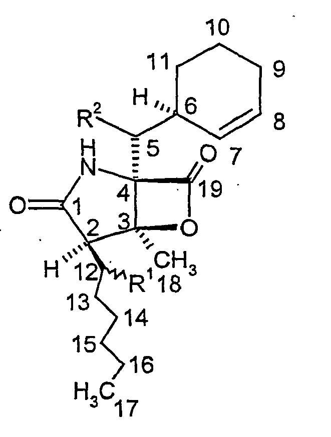 example 4, example 5: R⁸ = H, example 6: R⁸ = CH₃, example 7 (stereochemistry has not been determined by NMR).

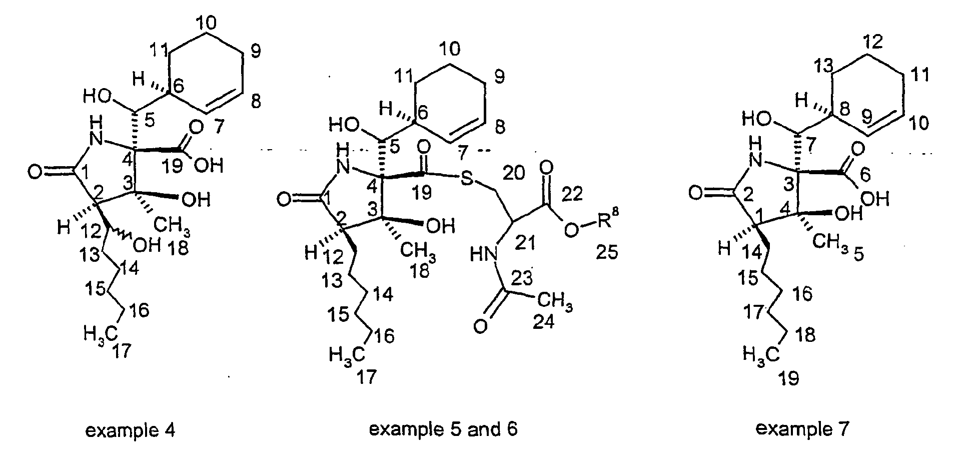

**Table 3a**

| Chemical shifts for the examples 1 to 3, as measured at 500 MHz, at 302 K in DMSO-d₆. | | | |
|---|---|---|---|
| **carbon** | **example 1** | **example 2** | **example 3** |
| C-1 | 174.1 | 172.3 | 170.8 |
| C-2 | 47.5 | 54.6 | 46.7 |
| C-3 | 86.4 | 76.0⁺⁺ | 86.1 |
| C-4 | 78.4 | 69.4⁺⁺ | 74.0 |
| C-5 | 69.3 | 75.0 | 32.5 |
| C-6 | 37.3 | 36.1 | 29.9 |
| C-7 | 128.6 | 127.9 | 130.9 |
| C-8 | 127.8 | 127.9 | 127.7 |
| C-9 | 25.2 | 24.2 | 24.0 |
| C-10 | 21.7 | 21.0 | 19.6 |
| C-11 | 26.1 | 29.6 | 28.2 |
| C-12 | 24.2 | 76.5 | 24.2 |
| C-13 | 26.8 | 31.5 | 26.5 |
| C-14 | 28.5 | 23.7 | 28.5 |
| C-15 | 30.8 | 30.1 | 30.9 |
| C-16 | 21.8 | 21.4 | 21.4 |
| C-17 | 13.8 | 13.4 | 13.3 |
| C-18 | 21.8 | 21.4 | 21.4 |
| C-19 | 169.1 | 170.9 | 170.4 |

| | | | |
|---|---|---|---|
| ⁺⁺ *These resonance assignments can be interchanged* | | | |

**Table 3b**

| Chemical shifts for the examples 4 to 6, as measured at 500 MHz, at 302 K in DMSO-d₆. | | | |
|---|---|---|---|
| **carbon** | **example 4** | **example 5** | **example 6** |
| C-1 | 176.5 | 178.9 | 178.9 |
| C-2 | 52.7 | 50.3 | 50.3 |
| C-3 | 80.1 | 80.6 | 80.6 |
| C-4 | 75.2 | 79.7 | 79.7 |
| C-5 | 73.6 | 75.3 | 75.3 |
| C-6 | 37.7 | 38.1 | 38.1 |
| C-7 | 128.6 | 129.3 | 129.3 |
| C-8 | 126.3 | 127.0 | 127.0 |
| C-9 | 23.5 | 24.3 | 24.3 |
| C-10 | 20.6 | 21.7 | 21.7 |
| C-11 | 23.6 | 26.8 | 26.8 |
| C-12 | 67.1 | 23.4 | 23.4 |
| C-13 | 33.5 | 27.7 | 27.7 |
| C-14 | 23.6 | 28.7 | 28.7 |
| C-15 | 30.5 | 31.1 | 31.1 |
| C-16 | 21.0 | 22.0 | 22.0 |
| C-17 | 13.1 | 13.9 | 13.6 |
| C-18 | 20.1 | 20.8 | 20.8 |
| C-19 | 171.2 | 201.2 | 201.2 |
| C-20 | - | 29.2 | 29.3 |
| C-21 | - | 51.0 | 51.4 |
| C-22 | - | 169.3 | 171.0 |
| C-23 | - | 22.2 | 169.3 |
| C-24 | - | 22.1 | 22.1 |
| C-25 | - | - | 51.8 |

**Table 3c**

| Chemical shifts for the example 7, as measured at 500 MHz, at 302 K in DMSO-d₆. | |
|---|---|
| **carbon** | **example 7** |
| C-1 | 50.5 |
| C-2 | 177.5 |
| C-3 | 75.5 |
| C-4 | 80.4 |
| C-5 | 20.3 |
| C-6 | - |
| C-7 | 74.6 |
| C-8 | 38.5 |
| C-9 | 129.7 |
| C-10 | 127.4 |
| C-11 | 24.4 |
| C-12 | 21.5 |
| C-13 | 26.5 |
| C-14 | 23.3 |
| C-15 | 27.9 |
| C-16 | 28.9 |
| C-17 | 31.0 |
| C-18 | 21.7 |
| C-19 | 13.8 |

**Table 4a**

| Chemical shifts for the examples 1 to 3, as measured at 500 MHz, at 302 K in DMSO-d₆. | | | |
|---|---|---|---|
| **proton** | **example 1** | **example 2** | **example** 3 |
| H-1 | - | - | - |
| H-2 | 2.41t t | 2.35 d | 2.55 t |
| H-3 | - | - | - |
| H-4 | - | - | - |
| H-5 | 3.65 m | 3.49 m | 1.76 m |
| H-6 | 2.29 m | 2.76 m | 2.27 m |
| H-7 | 5.81m | 5.91m | 5.45m |
| H-8 | 5.73 m | 5.70 m | 5.68 m |
| H-9 | 1.90,1.90 m | 1.93, 1.93 m | 1.92, 1.92m |
| H-10 | 1.40, 1.68m | 1.75, 1.49m | 1.46, 1.63m |
| H-11 | 1.23, 1.80 m | 1.59, 1.71 m | 1.33, 1.33 m |
| H-12 | 1.47, 1.58m | 4.68 m | 1.62, 1.50m |
| H-13 | 1.45, 1.54m | 1.58, 1.58m | 1.48, 1.48m |
| H-14 | 1.29, 1.29 m | 1.37, 1.37m | 1.31, 1.31m |
| H-15 | 1.28, 1.28m | 1.27, 1.27 m | 1.27, 1.27m |
| H-16 | 1.28, 1.28m | 1.29, 1.29m | 1.30, 1.30m |
| H-17 | 0.87 t | 0.88 t | 0.88 t |
| H-18 | 1.74 s | 1.35 s | 1.61 s |
| H-19 | - | - | - |
| H-N | 8.92s | 8.52 s | 8.98s |
| H-O-C-5 | 5.47 | 4.00 d | - |
| H-O-C-12 | - | 6.11 (tent.) | - |

**Table 4b**

| Chemical shifts for the examples 4 to 6, as measured at 500 MHz, at 302 K in DMSO-d₆. | | | |
|---|---|---|---|
| **proton** | **example 4** | **example 5** | **example 6** |
| H-1 | - | - | - |
| H-2 | 2.44 d | 2.47 t | 2.47 t |
| H-3 | - | - | - |
| H-4 | - | - . | - |
| H-5 | 3.68m | 3.78 m | 3.78 m |
| H-6 | 2.08 m | 2.13 m | 2.13 m |
| H-7 | 5.76 m | 5.79 m | 5.79 m |
| H-8 | 5.60 m | 5.64 m | 5.64 m |
| H-9 | 1.84 m | 1.87 m | 1.87 m |
| H-10 | 1.62,1.32 m | 1.61,1.33 m | 1.61,1.33 m |
| H-11 | 1.63,1.17 m | 1.64,1.09 m | 1.64,1.09 m |
| H-12 | 3.80 m | 1.46,1.37 m | 1.46,1.37 m |
| H-13 | 1.60 m | 1.50,1.35 m | 1.50,1.35 m |
| H-14 | 1.32,1.20 m | 1.24 m | 1.24 m |
| H-15 | 1.20 m | 1.25 m | 1.25 m |
| H-16 | 1.24 m | 1.27 m | 1.27 m |
| H-17 | 0.82 | 0.87 | 0.87 - |
| H-18 | 1.47 | 1.44 | 1.44 |
| H-19 | - | - | - |
| H-20 | - | 3.30,2.96 m | 3.24,3.00 m |
| H-21 | - | 4.36 m | 4.39 m |
| H-22 | - | - | - |
| H-23 | - | - | - |
| H-24 | - | 1.84 s | 1.84 s |
| H-25 | - | - | 3.64 s |

**Table 4c**

| Chemical shifts for the example 7, as measured at 500 MHz, at 302 K in DMSO-d₆. | |
|---|---|
| **proton** | **example 7** |
| H-1 | 2.45 t |
| H-5 | 1.46 s |
| H-7 | 3.77 d |
| H-8 | 2.12 m |
| H-9 | 5.82 m |
| H-10 | 5.64 m |
| H-11 | 1.88,1.88 m |
| H-12 | 1.36,1.66 m |
| H-13 | 1.67,1.25 m |
| H-14 | 1.34,1.44,m |
| H-15 | 1.33,1.51 m |
| H-16 | 1.25 m |
| H-17 | 1.25 m |
| H-18 | 1.26 m |
| H-19 | 0.86 t |
| N-H | 7.66 s |

### High resolution mass spectrometry

- example 1:: ESI- ; Mass found: 334.1977, calculated: 334.2014 (corresponding to a deviation of 4.1 mDa for the molecular formula C₁₉H₂₈NO₄)
- example 2:: ESI- ; Mass found: 350.1968, calculated: 350.1967 (corresponding to a deviation of 0.1 mDa for the molecular formula C₁₉H₂₈NO₅)
- example 3:: ESI+ ; Mass found: 276.2388, calculated 276.2327 (corresponding to a deviation of 6.1 mDa for the molecular formula C₁₈H₃₀NO). Here, only the fragment (M - CO₂) could be observed under the ESI conditions.
- example 4:: ESI+ ; Mass found: 368.2107, calculated 368.2073 (corresponding to a deviation of 3.3 mDa for the molecular formula C₁₉H₃₁NO₆
- example 5:: ESI- ; Mass found: 497.2322, calculated: 497.2321 (corresponding to a deviation of 0.0 mDa for the molecular formula C₂₄H₃₈N₂O₇S)
- example 6:: ESI- ; Mass found: 511.2594, calculated: 511.2478 (corresponding to a deviation of 11.6 mDa for the molecular formula C₂₅H₄₀N₂O₇S)
- example 7:: ESI+ ; Mass found: 354.2268, calculated 354.2280 (corresponding to a deviation of 1.3 mDa for the molecular formula C₁₉H₃₂NO₅).

### Single crystal X-ray structure analysis of example 1 and determination of the absolute configuration

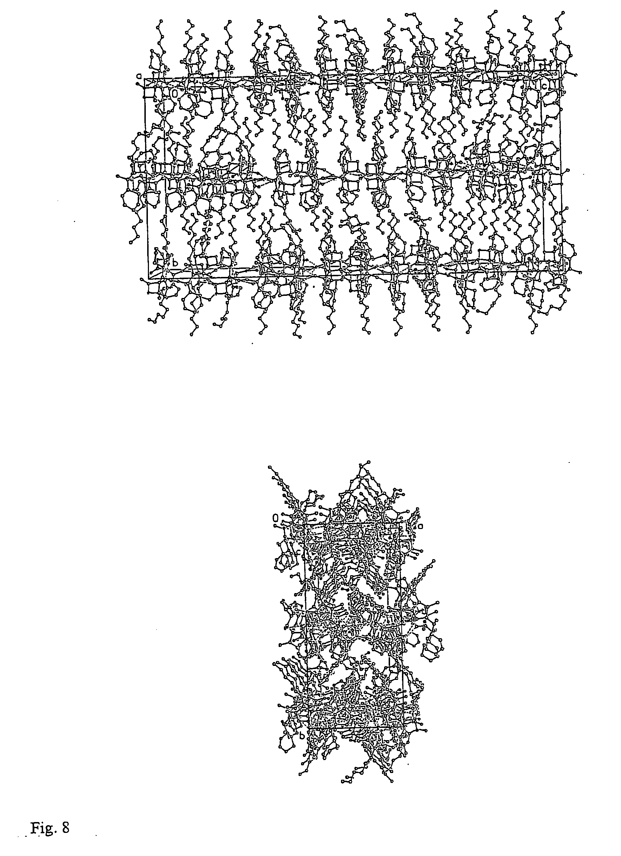

Several crystals of example 1 are crystallized by slow evaporation of a saturated solution of propanol/diacetone alcohol 97:3 at 46°C. A full data set is collected from a suitable crystal with the dimension 0.30 x 0.20 x 0.03 mm³ at -183.5°C using Cu_{Kα}-radiation as X-ray source. A structure proposal is obtained in an orthorhombic cell using the chiral space group *P*2₁2₁2₁ (see Fig. 7). The crystal cell has extreme large axes and contains 12 independent molecules of example 1 with identical chirality. All molecules show different conformations. The molecules are packed in polar and non polar layers (see Fig. 8). The single polar layers are connected two-dimensionally along hydrogen bondings. The absolute configuration of example 1 is thus determined with *R*(C2);*S*(C4);*R*(CS);*S*(C6);*S*(C7) obtaining a Flack Parameter of 0.0 with a standard deviation of 0.2 (H.-D. Flack, Acta Cryst., 1983, A39, 876-881). Expected values are 0 (within 3 esd's) for correct and +1 for inverted absolute structure.

numbering of chiral centers in X-ray structure of example 1:

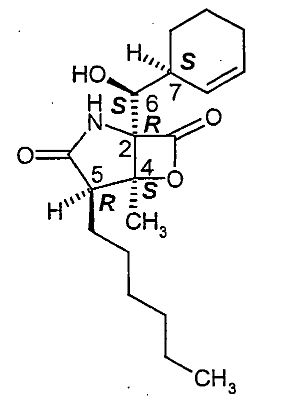

Data Collection for X-ray analsis: Measurements are made on a Bruker-Nonius diffractometer equipped with a Proteum CCD area detector, a FR591 rotating anode with Cu_{Kα} radiation, Montel mirrors as monochromator and a Kryoflex low temperature device (T = 90 K). The measurements are made in the range 4.69 to 54.33°. 205845 reflections are collected of which 26995 are unique (Rᵢₙₜ = 0.1073). Fullsphere data collection ω and ϕ scans. Programs used: Data collection Proteum V. 1.37 (Bruker-Nonius 2002), data reduction Saint Plus Version 1.6 (Bruker-Nonius 2002) and absorption correction SADABS V. 2.03 (2002).

Structure solution and refinement: SHELXTL Version 6.10 (Sheldrick 2000, University of Goettingen, Germany); 21119 Fo > 4sig(Fo), 2629 refmed parameters, *R*₁ = 0.0796, wR2 = 0.1892, Goodness of fit on F² = 1.083, Flack parameter 0.0(2), maximum residual electron density 0.464 (-0.314) e Å³.

Crystal Data: C₁₉H₂₉N₁O₄ x 12, Mᵣ = 335.26 (4023.17); orthorhombic; space group *P*2₁2₁2₁, *a* = 13.0624(2) Å, *b* = 29.0543(5) Å, c = 58.4559(11) Å, V =22178.2(7) Å³, Z *= 4, ρ_{cal}* = 1.205 Mg/m³, µ = 0.674 mm⁻¹.

### Preparing method of compounds

Liquid Chromatography - Mass spectroscopy (LC-MS): Micromass Platform LC with Shimadzu Phenomenex ODS column(4.6 mmφ X 30 mm) flushing a mixture of acetonitrile-water (9:1 to 1:9) at 1 ml/min of the flow rate. Mass spectra were obtained using electrospray (ES) ionization techniques.
Mass determination: Finnigan MAT MAT95
Melting points are uncorrected.
¹H NMR spectra were recorded using either Bruker DRX-300 (300 MHz for ¹H) spectrometer or Brucker 500 UltraShieled^{™} (500 MHz for 1H). Chemical shifts are reported in parts per million (ppm) with tetramethylsilane (TMS) as an internal standard at zero ppm. Coupling constant (J) are given in hertz and the abbreviations s, d, t, q, m, and br refer to singlet, doblet, triplet, quartet, multiplet, and broad, respectively.

TLC was performed on a precoated silica gel plate (Merck silica gel 60 F-254). Silica gel (WAKO-gel C-200 (75-150 µm)) was used for all column chromatography separations. All chemicals were reagent grade and were purchased from Sigma-Aldrich, Wako pure chemical industries, Ltd., Great Britain, Tokyo kasei kogyo Co., Ltd., Nacalai tesque, Inc., Watanabe Chemical Ind. Ltd., Maybridge plc, Lancaster Synthesis Ltd., Merck KgaA, Germany, or Kanto Chemical Co., Ltd.

All starting materials are commercially available or can be prepared using methods cited in the literature.

### Example 1

### 1-(Cyclohex-2-enyl-hydroxy-methyl)-4-hexyl-5-methyl-6-oxa-2-aza-bicyclo[3.2.0]heptane-3,7-dione

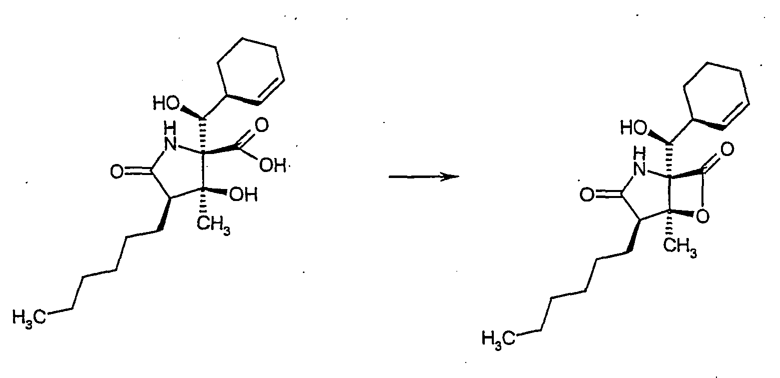

To a solution of 2-(Cyclohex-2-enyl-hydroxy-methyl)-4-hexyl-3-hydroxy-3-methyl-5-oxo-pyrrolidine-2-carboxylic acid (example 7) (130 mg, 0.37 mmol) in dichloromethane (10 ml) was added triethylamine (0.15 ml, 1.1 mmol) and BOPCl (140 mg, 0.55 mmol) at rt. After being stirred for 1 hour, saturated solution of sodium hydrogencarbonate (20 ml) was added there and then the organic layer was extracted with ethyl acetate, washed with brine and dried over magnesium sulfate. After concentration, the residue was purified by column chromatography (hexane/ethyl acetate=3/1-1/1) to give the product (98 mg, 79 %).
mp: 157°C;
LCMS (4min method): Rₜ = 2.56 min, m/z = 336 (M+H)⁺;
¹H-NMR (500Mz, DMSO-d₆): δ = 0.87 (3H, t, *J*= 7.0 Hz), 1.10-1.70 (13H, m), 1.74 (3H, s), 1.82 (1H, m), 1.92 (2H, m), 2.29 (1H, m), 2.41 (1H, d, *J*= 5.8 Hz), 3.66 (1H, t, *J*= 8.9 Hz), 5.49 (1H, d, *J*= 7.9 Hz), 5.70 (1H, m), 5.80 (1H, d, *J*= 11.5 Hz), 8.91 (1H, s).

### Example 2

### 1-(Cyclohex-2-enyl-hydroxy-methyl)-4-(1-hydroxy-hexyl)-5-methyl-6-oxa-2-aza-bicyclo[3.2.0]-heptane-3,7-dione

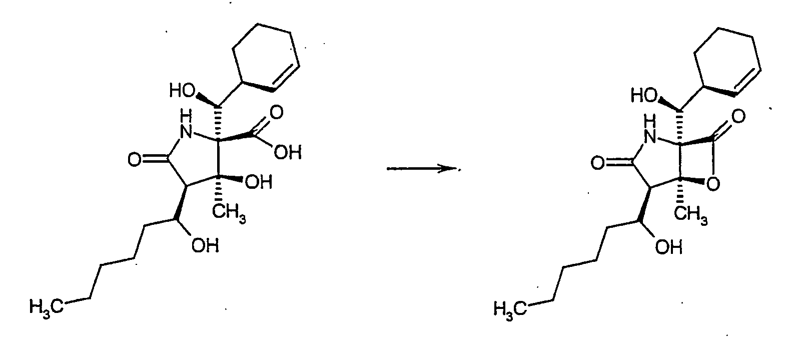

To a solution of 2-(Cyclohex-2-enyl-hydroxy-methyl)-3-hydroxy-4-(1-hydroxy-hexyl)-3-methyl-5-oxo₋pyrrolidine-2-carboxylic acid (example 4) (239 mg, 0.65 mmol) in dichloromethane (14 ml) was added triethylamine (0.27 ml, 1.9 mmol) and BOPCl (247 mg, 0.97 mmol) at rt. After being stirred for 1 hour, saturated solution of sodium hydrogencarbonate (20 ml) was added there and then the organic layer was extracted with ethyl acetate, washed with brine and dried over magnesium sulfate. After concentration, the residue was purified by column chromatography (hexane/ethyl acetate=3/1-1/1) to give the product (92 mg, 40 %).
mp: 144°C;
LCMS (4min method): Rₜ = 2.55 min, m/z = 352 (M+H)⁺;
¹H-NMR (500Mz, DMSO-d₆): δ = 0.87 (3H, t, *J*= 7.0 Hz), 1.17-1.33 (6H, m), 1.46-1.52 (3H, m), 1.77 (3H, s), 1.54-1.86 (3H, m), 1.92 (2H, m), 2.29 (1H, m), 2.57 (1H, d, *J*= 5.7 Hz), 3.65 (1H, t, *J* = 8.8 Hz), 3.92 (1H, m), 4.77 (1H, d, J= 3.8 Hz), 5.48 (1H, d, *J*= 7.9 Hz), 5.72 (1H, m), 5.80 (1H, d, *J*= 10.4 Hz), 9.07 (1H, s).

### Example 8

### 1-(Cyclohexyl-hydroxy-methyl)-4-hexyl-5-methyl-6-oxa-2-aza-bicyclo[3.2.0]heptane-3,7-dione

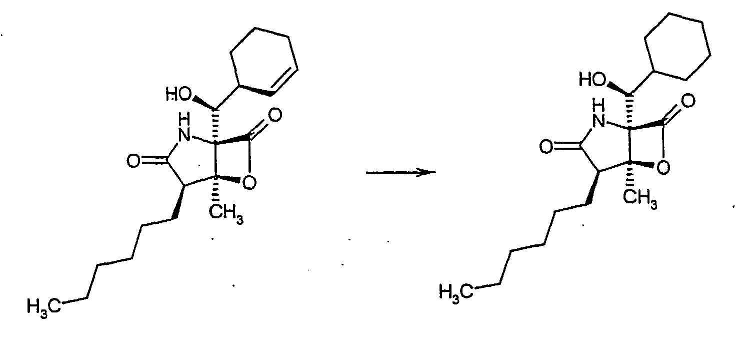

To a suspension of 1-(Cyclohex-2-enyl-hydroxy-methyl)-4-hexyl-5-methyl-6-oxa-2-aza-bicyclo-[3.2.0]heptane-3,7-dione (example 1) (100 mg, 0.3 mmol) and 10 % Pd-C (5 mg) in dichloromethane (2 ml) was charged hydrogen carefully. After being stirred at rt for 3 hours, the catalyst was removed by filtration. The residue was purified by column chromatography (hexane/ethyl acetate=4/1) to give the product (50 mg, 50 %).
mp: 167°C;
LCMS (4min method): Rₜ = 2.73 min, m/z = 338 (M+H)⁺;
¹H-NMR (500Mz, DMSO-d₆): δ = 0.87 (3H, t, *J*= 6.9 Hz), 0.90-1.35 (12H, m), 1.73 (3H, s), 1.40-1.86 (9H, m), 2.40 (1H, t, *J*= 7.6 Hz), 3.67 (1H, t, *J*= 7.9 Hz), 5.23 (1H, d, *J*= 7.9 Hz), 8.85 (1H, s).

### Example 9

### (1S)-Cyclohex-2-en-1-yl[(1R,4R,5S)-4-hexyl-5-methyl-3,7-dioxo-6-oxa-2-azabicyclo[3.2.0]hept-1-yl]methyl acetate

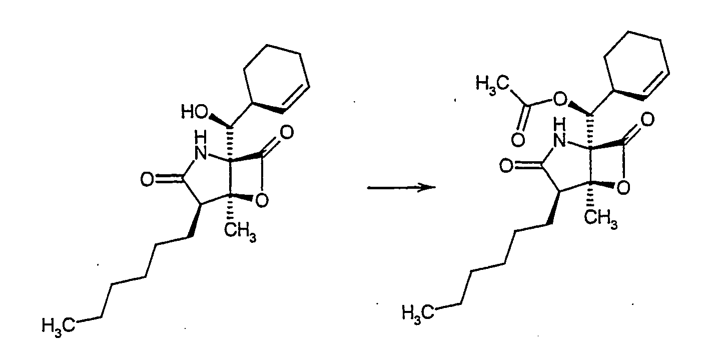

A mixture of (1*R*,4*R*,5*S*)-1-[(1*S*)-cyclohex-2-en-1-yl(hydroxy)methyl]-4-hexyl-5-methyl-6-oxa-2-azabicyclo[3.2.0]heptane-3,7-dione (example 1) (8.30 mg, 0.025 mmol) and acetic anhydride (2.78 mg, 0.027 mmol) in pyridine (0.002 ml) was stirred for 18 hours at room temperature. After this, the mixture was diluted with toluene and concentrated under reduced pressure to give (1*S*)-cyclohex-2-en-1-yl[(1*R*,4*R*,5*S*)-4-hexyl-5-methyl-3,7-dioxo-6-oxa-2-azabicyclo[3.2.0]hept-1-yl]-methyl acetate (9.00 mg, 96%).
MS: m/z = 378 (M+H)⁺;
¹H-NMR (500 MHz, DMSO-d₆): δ = 0.87 (3H, t, *J*= 7.0 Hz), 1.22-1.34 (6H, m), 1.40-1.85 (8H, m), 1.70 (3H, s), 1.85-2.02 (3H, m), 2.07 (3H, s), 2.67 (1H, dd, *J*= 8.0, 5.5 Hz), 5.19 (1H, d, *J*= 8.5 Hz), 5.41 (1H, dd, *J*= 10.5, 2.1 Hz), 5.74 (1H, m), 8.17 (1H, s).

### Example 10

### 2-(Cyclohex-2-enyl-hydroxy-methyl)-4-hexyl-3-hydroxy-3-methyl-5-oxo-pyrrolidine-2-carbothioic acid S-benzyl ester

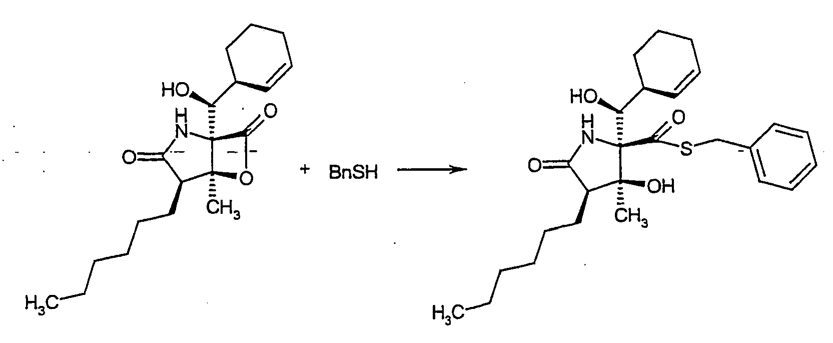

To a solution of 1-(Cyclohex-2-enyl-hydroxy-methyl)-4-hexyl-5-methyl-6-oxa-2-aza-bicyclo-[3.2.0]heptane-3,7-dione (example 1) (30 mg, 0.09 mmol) and triethylamine (37 µl, 0.27 mmol) in dichloromethane (2 ml) was added benzyl hydrosulfide (0.1 ml) at rt. After being stirred for 4 hours, the mixture was purified by column chromatography (hexane-only-hexane/ethyl acetate=3/1-1/1) to give the product, which was washed with Hexane to give a white solid (25 mg, 61 %).
mp: 138°C;
LCMS (4min method): Rₜ = 2.92 min, m/z = 460 (M+H)⁺;
¹H-NMR (500Mz, DMSO-d₆): δ = 0.86 (3H, t, *J*= 6.9 Hz), 0.98 (1H, m), 1.46 (3H, s), 1.12-1.58 (13H, m), 1.81 (2H, m), 2.07 (1H, m), 2.43 (1H, m), 3.79 (1H, t, *J*= 6.5 Hz), 4.00 (1H, d, *J*= 13.8 Hz), 4.09 (1H, d, *J*= 13.8 Hz), 4.84 (1H, s), 5.00 (1H, d, *J*= 7.6 Hz), 5.60 (1H, m), 5.76 (1H, d, *J* =12.0 Hz), 7.18-7.32 (5H, m), 8.14 (1H, s).

### Example 11

### 2-(Cyclohex-2-enyl-hydroxy-methyl)-4-hexyl-3-hydroxy-3-methyl-5-oxo-pyrrolidine-2-carbothioic acid S-(2-acetylamino-ethyl) ester

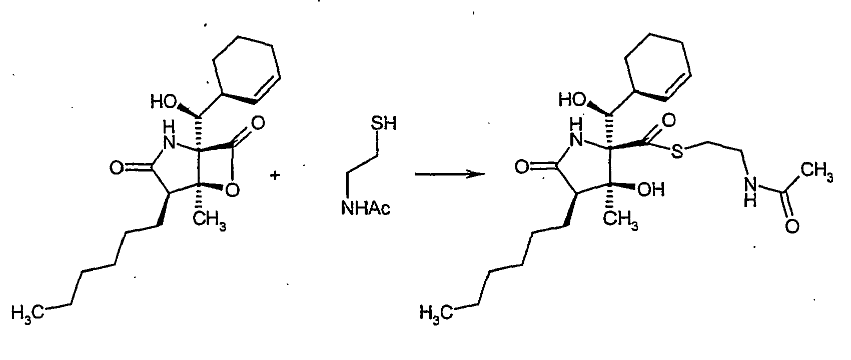

To a solution of 1-(Cyclohex-2-enyl-hydroxy-methyl)-4-hexyl-5-methyl-6-oxa-2-aza-bicyclo-[3.2.0]heptane-3,7-dione (example 1) (20 mg, 0.06 mmol) and triethylamine (25 µl, 0.27 mmol) in dichloromethane (1 ml) was added thiol (0.05 ml) at rt. After being stirred for 1 hour, the mixture was purified by column chromatography (hexane-only-hexane/ethyl acetate=3/1-1/1) to give the product, which was washed with hexane to give a white solid (10 mg, 37 %).
mp: 82°C;
LCMS (4min method): Rₜ = 2.38 min, m/z = 455 (M+H)⁺;
¹H-NMR (500Mz, DMSO-d₆): δ = 0.86 (3H, t, *J*= 6.8 Hz), 1.09 (1H, m), 1.44 (3H, s), 1.19-1.55 (11H, m), 1.62 (2H, m), 1.79 (3H, m), 1.85 (2H, m), 2.13 (1H, m), 2.44 (1H, m), 2.82 (2H, m), 3.13 (2H, m), 3.79 (1H, t, *J* = 7.0 Hz), 4.76 (1H, s), 5.02 (1H, d, *J*= 7.6 Hz), 5.63 (1H, m), 5.79 (1H, d, *J* = 10.1 Hz), 7.93 (1H, m), 8.18 (1H, s).

### Example 12

### 3-[2-(Cyclohex-2-enyl-hydroxy-methyl)-4-hexyl-3-hydroxy-3-methyl-5-oxo-pyrrolidine-2-carbonylsulfanyl]-propionic acid methyl ester

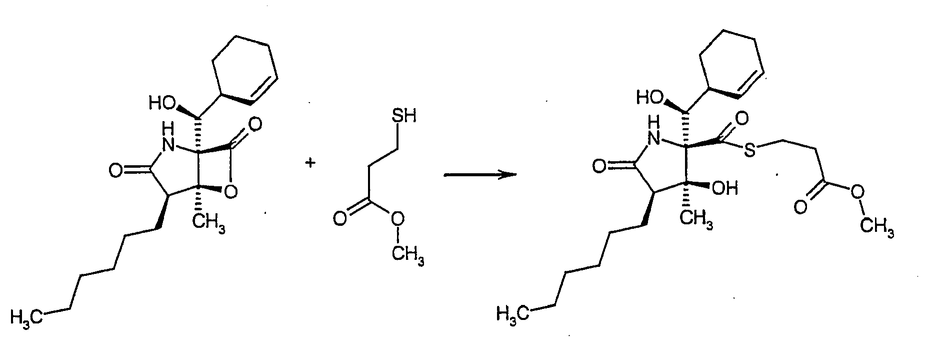

To a solution of 1-(Cyclohex-2-enyl-hydroxy-methyl)-4-hexyl-5-methyl-6-oxa-2-aza-bicyclo-[3.2.0]heptane-3,7-dione (example 1) (20 mg, 0.06 mmol) and triethylamine (25 µl, 0.18 mmol) in dichloromethane (1 ml) was added thiol (0.05 ml) at rt. After being stirred for 4 hours, the mixture was purified by column chromatography (hexane-only-hexane/ethyl acetate=3/1-1/1) to give the product, which was washed with hexane to give a white solid (10 mg, 37 %).
mp: 64°C;
LCMS (4min method): Rₜ = 2.64 min, m/z = 456 (M+H)⁺;
¹H:-NMR (500Mz, DMSO-d₆): δ = 0.86 (3H, t, *J*= 6.9 Hz), 1.09 (1H, m), 1.43 (3H, s), 1.15-1.54 (11H, m) 1.61 (2H, m), 1.86 (2H, m), 2.12 (1H, m), 2.44 (1H, t, *J*= 5.9 Hz), 2.56 (2H, t, *J*= 6.8 Hz), 2.95 (2H, t, *J*= 7.1 Hz), 3.60 (3H, s), 3.77 (1H, t, *J*= 7.1 Hz), 4.76 (1H, s), 5.01 (1H, d, *J*= 7.7 Hz), 5.63 (1H, m), 5.78 (1H, d, *J* = 11.9.Hz), 8.18 (1H, s).

### Example 13

### 2-(Cyclohex-2-enyl-hydroxy-methyl)-4-hexyl-3-hydroxy-3-methyl-5-oxo-pyrrolidine-2-carbothioic acid S-cyclohexyl ester

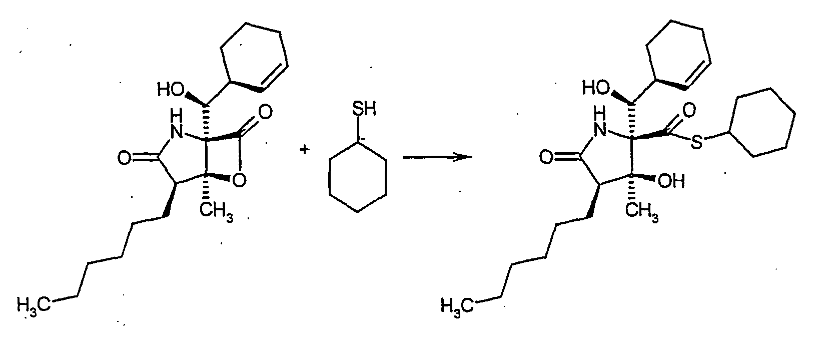

To a solution of 1-(Cyclohex-2-enyl-hydroxy-methyl)-4-hexyl-5-methyl-6-oxa-2-aza-bicyclo-[3.2.0]heptane-3,7-dione (example 1) (20 mg, 0.06 mmol) and triethylamine (83 µl, 0.6 mmol) in dichloromethane (1 ml) was added thiol (0.1 ml) at rt. After being stirred for 40 hours, the mixture was purified by column chromatography (hexane-only-hexane/ethyl acetate=3/1-1/1) to give the product, which was washed with hexane to give a white solid (16 mg, 59 %).
mp: 85°C;
LCMS (4min method): Rₜ = 3.04 min, m/z = 452 (M+H)⁺;
¹H-NMR (500Mz, DMSO-d₆): δ = 0.86 (3H, t, *J*= 7.0 Hz), 1.43 (3H, s), 1.09-1.56 (18H, m), 1.56-1.73 (4H, m), 1.74-1.89 (4H, m), 2.15 (1H, m), 2.42 (1H, m), 3.36 (1H, m), 3.79 (1H, t, *J* = 6.6 Hz), 4.69 (1H, s), 4.94 (1H, d, *J*= 7.6 Hz), 5.64 (1H, m), 5.78 (1H,d, *J*= 10.1 Hz), 8.02 (1H, s).

### Example 14

### 2-(Cyclohex-2-enyl-hydroxy-methyl)-3-hydroxy-4-(1-hydroxy-heXyl)-3-methyl-5-oxo-pyrrolidine-2-carbothioic acid S-benzyl ester

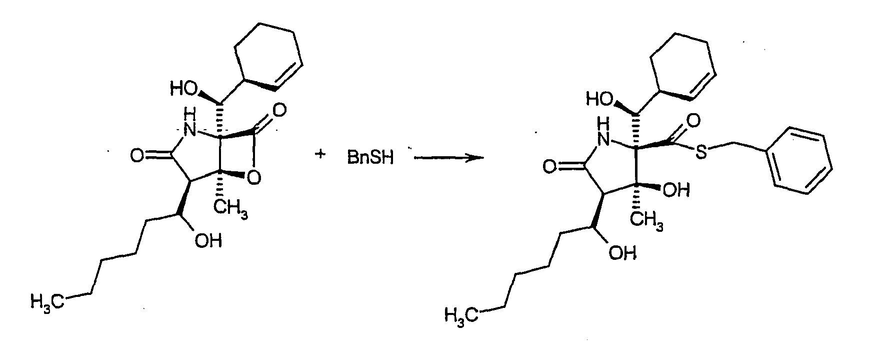

To a solution of 1-(Cyclohex-2-enyl-hydroxy-methyl)-4-(1-hydroxy-hexyl)-5-methyl-6-oxa-2-aza-bicyclo[3.2.0]heptane-3,7-dione (example 2) (30 mg, 0.09 mmol) and triethylamine (36 µl, 0.26 mmol) in dichloromethane (2 ml) was added benzyl hydrosulfide (0.05 ml) at rt. After being stirred for 4 hours, the mixture was purified by column chromatography (hexane-only-hexane/ethyl acetate=3/1-1/1) to give the product, which was washed with hexane to give a white solid (17 mg, 41 %).
mp: 123°C;
LCMS (4min method): Rₜ = 2.84 min, m/z = 476 (M+H)⁺;
¹H-NMR (500Mz, DMSO-d₆): δ = 0.87 (3H, t, *J*= 7.0 Hz), 0.94 (1H, m), 1.49 (3H, s), 1.14-1.56 (9H, m), 1.71 (2H, m), 1.81 (2H, m), 2.08 (1H, m), 2.48 (1H, m), 3.77 (1H, t, *J* = 7.3 Hz), 3.82 (1H, m), 4.01 (1H, d, *J*= 13.8 Hz), 4.11 (1H, d, *J*= 13.9 Hz), 5.11 (1H, d, *J*= 7.3 Hz), 5.20 (1H, d, *J*= 2.9 Hz), 5.48 (1H, s), 5.61 (1H, m), 5.75 (1H, d, *J*=10.7 Hz), 7.17-7.32 (5H, m), 8.45 (1H, s).

### Example 15

### 2-(Cylohex-2-enyl-hydroxy-methyl)-3-hydroxy-4-(1-hydroxy-hexyl)-3-methyl-5-oxo-pyrrolidine-2-carbothioic acid S-(2-acetylamino-ethyl) ester

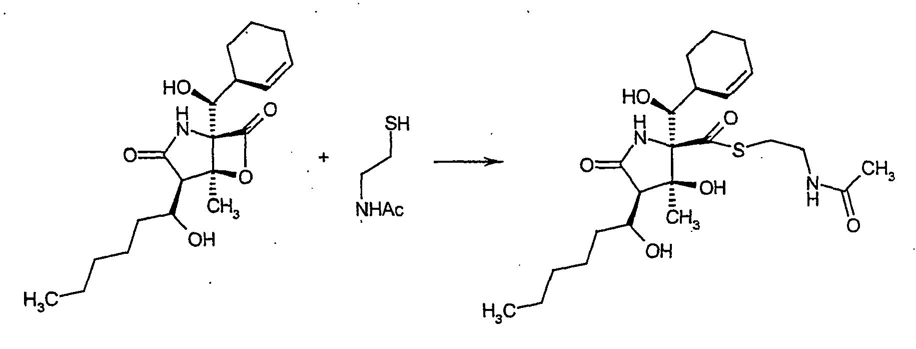

To a solution of 1-(Cyclohex-2-enyl-hydroxy-methyl)-4-(1-hydroxy-hexyl)-5-methyl-6-oxa-2-aza-bicyclo[3.2.0]heptane-3,7-dione (example 2) (30 mg, 0.09 mmol) and triethylamine (36 µl, 0.26 mmol) in dichloromethane (2 ml) was added thiol (0.05 ml) at rt. After being stirred for 1 hour, the mixture was purified by column chromatography (hexane-only-hexane/ethyl acetate=3/1-1/1) to give the product, which was washed with hexane to give a white solid (25 mg, 62 %).
mp: 80°C;
LCMS (4min method): Rₜ = 2.19 min, m/z = 471 (M+H)⁺;
1H-NMR (500Mz, DMSO-d₆): δ = 0.87 (3H, t, *J*= 6.3 Hz), 1.48 (3H, s), 1.01-1.75 (12H, m), 1.79 (3H, s), 1.87 (2H, m); 2.14 (1H, m), 2.48 (1H, m), 2.84 (2H, t, *J*= 7.0 Hz), 3.13 (2H, m), 3.77 (1H, t, *J* = 7.0 Hz), 3.82 (1H, m), 5.11 (1H, d, *J* = 7.3 Hz), 5.19 (1H, m), 5.41 (1H, s), 5.64 (1H, m), 5.77 (1H, d, *J*= 10.1 Hz), 7.93 (1H, m), 8.49 (1H, s).

### Example 16

### [2-(Cyclohex-2-enyl-hydroxy-methyl)-3-hydroxy-4-(1-hydroxy-hexyl)-3-methyl-5-oxo-pyrrolidine-2-carbonylsulfanyl]-acetic acid methyl ester

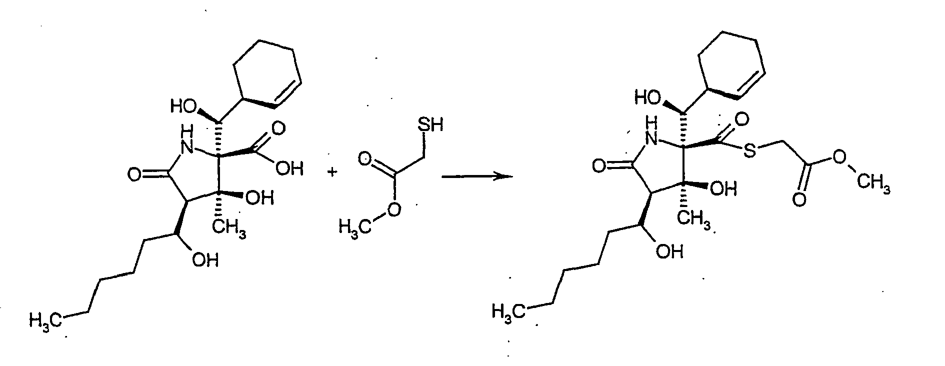

To a solution of 2-(Cylohex-2-enyl-hydroxy-methyl)-3-hydroxy-4-(1-hydroxy-hexyl)-3-methyl-5-oxo-pyrrolidine-2-carboxylic acid (example 2) (50 mg, 0.14 mmol) in THF (3 ml) was added triethylamine (57 µl, 0.4 mmol), thiol (0.05 ml) and BOPCl (52 mg, 0.2 mmol) at rt. After being stirred for 3 hours, a saturated solution of sodium hydrogencarbonate (10 ml) was added there and then the organic layer was extracted with ethyl acetate washed with brine and dried over magnesium sulfate. The residue was purified by column chromatography (hexane/ethyl acetate=3/1-1/1) to give the product (21 mg, 34%).
mp: 75°C;
LCMS (4min method): Rₜ = 2.46 min, m/z =458 (M+H)⁺;
¹H-NMR (500Mz, DMSO-d₆): δ = 0.86 (3H, t, *J*= 6.9 Hz), 1.02 (1H, m), 1.19-1.39 (7H, m), 1.46 (3H, s), 1.56-1.76 (4H, m), 1.86 (2H, m), 2.17 (1H, m), 2.48 (1H, m), 3.61 (3H, s), 3.68 (2H, s), 3.74 (1H, t, *J*= 7.2 Hz), 3.80 (1H, m), 5.13 (1H, d, *J*= 7.3 Hz), 5.17 (1H, d, *J*= 2.8 Hz), 5.45 (1H, s), 5.64 (1H, m), 5.78 (1H, d, *J*= 10.4 Hz), 8.57 (1H, s).

### Example 17

### 2-(Cyclohexyl-hydroxy-methyl)-4-hexyl-3-hydroxy-3-methyl-5-oxo-pyrrolidine-2-carbothioic acid S-benzyl ester

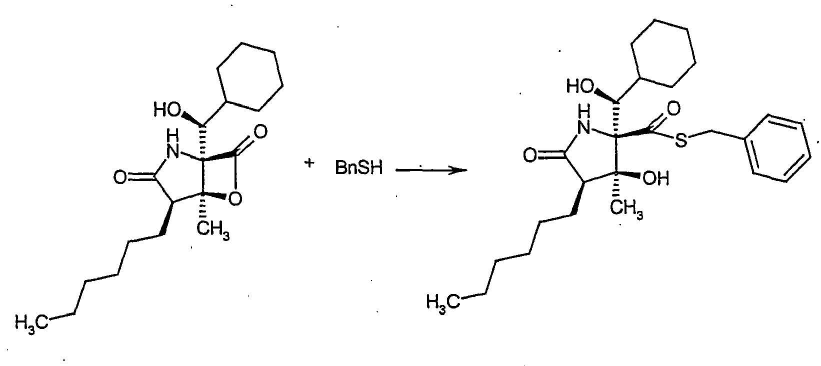

To a solution of 1-(Cyclohexyl-hydroxy-methyl)-4-hexyl-5-methyl-6-oxa-2-aza-bicyclo[3.2.0]-heptane-3,7-dione (example 8) (20 mg, 0.06 mmol) and triethylamine (25 µl, 0.18 mmol) in dichloromethane (2 ml) was added benzyl hydrosulfide (0.05 ml) at rt. After being stirred for 18 hours, the mixture was purified by column chromatography (hexane-only-hexane/ethyl acetate=3/1-1/1) to give the product, which was washed with hexane to give a white solid (15 mg, 55%).
mp: 181°C;
LCMS (4min method): Rₜ = 2.89 min, m/z = 462 (M+H)⁺;
¹H-NMR (500Mz, DMSO-d₆): δ = 0.86 (3H, t, *J*= 7.0 Hz), 0.87-1.05 (4H, m), 1.20-1.50 (15H, m), 1.45 (3H, s), 1.56 (1H, m), 1.79 (1H, m), 2.42 (1H, t, *J*= 6.3 Hz), 3.75 (1H, dd, *J*= 5.4, 7.0 Hz), 4.00 (1H, d, *J*= 13.9 Hz), 4.10 (1H, d, *J*= 13.9 Hz), 4.79-7.83 (2H, m), 7.20-7.30 (5H, m), 7.85 (1H, s).

### Example 18

### 3-[2-(Cyclohexyl-hydroxy-methyl)-4-hexyl-3-hydroxy-3-methyl-5-oxo-pyrrolidine-2-carbonyl-sulfanyl]-propionic acid methyl ester

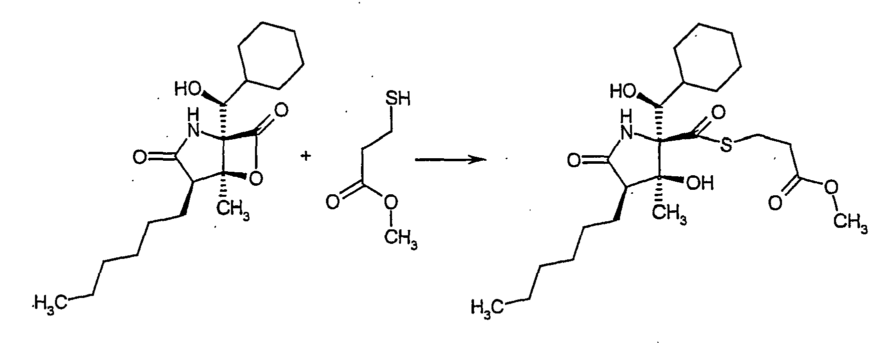

To a solution of 1-(Cyclohexyl-hydroxy-methyl)-4-hexyl-5-methyl-6-oxa-2-aza-bicyclo[3.2.0]-heptane-3,7-dione (example 8) (20 mg, 0.06 mmol) and triethylamine (25 µl, 0.18 mmol) in dichloromethane (1 ml) was added thiol (0.05 ml) at rt. After being stirred for 18 hours, the mixture was purified by column chromatography (hexane-only-hexane/ethyl acetate=3/1-1/1) to give the product, which was washed with hexane to give a white solid (14 mg, 52 %).
mp:132°C;
LCMS (4min method): Rₜ = 2.66 min, m/z =458 (M+H)⁺;
¹H-NMR (500Mz, DMSO-d₆): δ = 0.86 (3H, t, *J*= 6.9 Hz), 1.43 (3H, s), 0.85-1.90 (21H, m), 2.41 (1H, m), 2.55 (2H, m), 2.96 (2H, m), 3.23 (3H, s), 3.73 (1H, t, *J*= 5.6 Hz), 4.73 (1H, s), 4.83 (1H, d, *J*= 7.3 Hz), 7.95 (1H, s).

### B. Evaluation of Physiological activity

The *in vitro* effect of the compounds according to the invention can be demonstrated in the following assays:

### HTS Assay

The test compounds are diluted 6-fold with 50 mM Tris-HCl (pH8.0), 0.5 mM EDTA, 0.005% TritonX-100 and 0.075% SDS containing 150 µM Suc-Leu-Leu-Val-Tyr-MCA.

To each well of a 1536 well black plate 2 µl of a diluted compound solution is pipetted and then 3 µl of 0.5 µg/ml 20S proteasome (mammalian, AFFINITI, Exeter, U. K.) dissolved in 50 mM Tris-HCl (pH8.0), 0.5 mM EDTA and 0.005% TritonX-100 is added. After 1 hour incubation at room temperature, the reaction is terminated by addition of 3 µl of 13 µM Z-Leu-Leu-Leu-H and the fluorescence intensity is measured at λex 355 nm and λem 460 nm on a ARVO multilabel counter (Perkin Elmer, Tokyo, Japan).

### Proteasome inhibition assay

The test compound is diluted at various concentrations in 2.5% DMSO in a polypropylene 96 well plate. As an internal control, MG-132 (Cat.#3175-v; Peptide Institute; Osaka, Japan) is diluted using the same procedure as for the test compound. The diluted working solution (10 µl/well) is transferred into a polypropylene 96 well plate. The assay buffer consists of 50 mM Tris-HCl (pH8.0), 0.5 mM EDTA, 0.005% TritonX-100, 0.005% SDS, prepared as a stock solution at 10x concentration. The peptide substrate (Suc-Leu-Leu-Val-Tyr-MCA; 3120v; Peptide Institute; Osaka, Japan) is stored at 10 mM in 100% DMSO. The peptide substrate is diluted at 125 µM in 1.25x concentration of the assay buffer and 40 µl of the substrate solution is added to the compounds solution. The compound and the substrate are preincubated for 10 min at room temperature. Then the mixture of the compound and the substrate (10 µl/well) is transferred to a black non-coated 384 well assay plate (Nunc) and autofluorescence emission is measured at 460 nm (λex, 360 nm) by using a ARVO fluorescence plate leader (Perkin Elmer, Tokyo, Japan).

Human red blood cell S20 proteasome are obtained from Affinity research products Ltd (Cat#PW8720; Exeter, UK) and stored at -80°C. The proteasome is diluted 1 in 1000 with 1x concentration of the assay buffer and 10 µl is added to the substrates and the inhibitor mixture in the plate. The proteolytic reaction is performed at room temperature. The fluorescence emission is continuously measured for 90 min. IC₅₀ values of the compounds are determined at initial velocity of the reaction. Selected data are given in table A.

**Table A**

| **example** | **IC₅₀ [nM]** |
|---|---|
| **1** | 1 |
| **4** | 305 |
| **12** | 0.2 |
| **17** | 106 |

### Chymotrypsin assay

The test compound is diluted at various concentrations in 2.5% DMSO in a polypropylene 96 well plate. As an internal control, chymostatin (Cat.#4063; Peptide Institute; Osaka, Japan) is diluted using the same procedure as for the test compound. The diluted working solution (10 µl/well) was transferred into a polypropylene 96 well plate. The assay buffer consists of 50 mM TES (pH8.0), 10 mM CaCl₂, 0.1 mg/ml BSA, prepared as a stock solution at 10x concentration. The peptide substrate (Suc-Leu-Leu-Val-Tyr-MCA; 3120v; Peptide Institute; Osaka, Japan) is stored at 10 mM in 100% DMSO. The peptide substrate is diluted at 50 µM in 1.25x concentration of the assay buffer and 40 µl of the substrate solution is added to the compounds solution. The compound and the substrate are preincubated for 10 min at room temperature. Then the mixture of the compound and the substrate (10 µl/well) is transferred to a black non-coated 384 well assay plate (Nunc) and autofluorescence emission is measured at 460 nm (λex, 360 nm) by using a ARVO fluorescence plate leader (Perkin Elmer, Tokyo, Japan).

Human chymotrypsin is obtained from Calbiochem (Cat.#230900) and diluted at 0.5 mg/ml in 50% glycerol stored at -20°C. The chymotrypsin stock solution is diluted at 18 ng/ml in 1x concentration of the assay buffer and 10 µl is added to the substrates and the inhibitor mixture in the plate: The proteolytic reaction is performed at room temperature. The fluorescence emission is continuously measured for 60 min. IC₅₀ values of the compounds are determined at initial velocity of the reaction.

### Trypsin assay

The test compound is diluted at various concentrations in 2.5% DMSO in a polypropylene 96 well plate. As an internal control, leupeptin (Cat.#4041-v; Peptide Institute; Osaka, Japan) is diluted using the same procedure as for the test compound. The diluted working solution (10 µl/well) is transferred into a polypropylene 96 well plate. The assay buffer consists of 50 mM Tris-HCl (pH8.0), 150 mM NaCl, 1 mM CaCl₂, 0.1 mg/ml BSA 50 mM, prepared as a stock solution at 10x concentration. The peptide substrate (Boc-Gln-Ala-Arg-MCA; 3135-v Peptide Institute; Osaka, Japan) is stored at 1 mM in 100% DMSO. The peptide substrate is diluted at 15 µM in 1.25x concentration, of the assay buffer and 40 µl of the substrate solution is added to the compounds solution. The compound and the substrate are preincubated for 10 min at room temperature. Then the mixture of the compound and the substrate (10 µl/well) is transferred to a black non-coated 384 well assay plate (Nunc) and autofluorescence emission is measured at 460 nm (λex, 360 nm) by using a ARVO fluorescence plate leader (Perkin Elmer, Tokyo, Japan).

Trypsin is obtained from Calbiochem and diluted at 1 mg/ml in 1 mM HCl and stored at -20°C. The trypsin stock solution is diluted at 1 ng/ml in 1x concentration of the assay buffer and 10 µl is added to the substrates and the inhibitor mixture in the plate. The proteolytic reaction is performed at room temperature. The fluorescence emission is continuously measured for 60 min. IC₅₀ values of the compounds are determined at initial velocity of the reaction.

### TNFα-induced RANTES production in A549 cells

The A549 human lung epithelium cell line (ATCC #CCL-885) is maintained in Dulbecco's modified Eagle's medium (D-MEM, Nikken Biomedical Institute) supplemented with 10% FCS (Gibco), 100 U/ml penicillin, 100 µg/ml streptomycin and 2 mM glutamine. A549 cells (4x10⁴ cells in 80 µl/well) are treated in a 96-well flat-bottom tissue culture plate for 1 h with vehicle (0,1 % DMSO) or test compounds. Then the cells are stimulated with 100 ng/ml TNF-α for 24 h. The concentration of RANTES in the supernatants, which are collected after 24 h, is determined using a quantitative sandwich Fluorescent immunoassay technique following the manufacturer's recommendations (R&D Systems, Oxon, UK).

### TNFα-induced IκBα degradation in A549 cells

Sub-confluent A549 cells growing in 6-well plates are pretreated with various concentration of inhibitor or vehicle (0.1% DMSO) for 30 min at 37°C. Then, the cells are left untreated or stimulated with 10 ng/ml TNF-α for the indicated.period of time. The cells are washed with cold PBS twice and lysed by 100 µl SDS-PAGE sample buffer on ice. The cell lysates are briefly sonicated, centrifuged and the supernatants are subjected to SDS-PAGE and Western Blot analysis by using anti-IκBα (New England Biolabs #9242) according to manufacturer's recommendations.

### Inhibition of MDA MB 231 and H460 tumor cell proliferation

Cells (3000) plated in 96-well assay plate at 3000 cells/well in complete media with 10% Fetal Calf Serum and incubated 24hrs at 37°C. At 24 hrs after plating, compounds were added at final concentration range of between 10µM with serial dilutions to 10nM at a final DMSO concentration of 0.1 %. Cells incubated for 72hrs at 37°C in complete growth media after compound addition. Using the Promega Cell TiterGlo ATP Luminescent assay kit (Promega Corp), the number of viable cells/well is determined via measurement of luminescent signal based on amount of cellular ATP as an indirect measure of cell number. Values read at 72hrs after incubation with test compounds are subtracted from Day 0 values. IC₅₀ values determined with Analyze 5 program. Average Signal/Noise across cell types = 3 - 5 fold.

### C. Operative examples relating to pharmaceutical compositions

The compounds according to the invention can be converted into pharmaceutical preparations as follows:

### Tablet

### Composition

100 mg of the compound of example 1, 50 mg of lactose (monohydrate), 50 mg of maize starch (native), 10 mg of polyvinylpyrrolidone (PVP 25) (from BASF, Ludwigshafen, Germany) and 2 mg of magnesium stearate.

Tablet weight 212 mg, diameter 8 mm, curvature radius 12 mm.

### Preparation

The mixture of active component, lactose and starch is granulated with a 5% solution (m/m) of the PVP in water. After drying, the granules are mixed with magnesium stearate for 5 min. This mixture is moulded using a customary tablet press (tablet format, see above). The moulding force applied is typically 15 kN.

### Orally administrable suspension

### Composition

1000 mg of the compound of example 1, 1000 mg of ethanol (96%), 400 mg of Rhodigel (xanthan gum from FMC, Pennsylvania, USA) and 99 g of water.

A single dose of 100 mg of the compound according to the invention is provided by 10 ml of oral suspension.

### Preparation

The Rhodigel is suspended in ethanol and the active component is added to the suspension. The water is added with stirring. Stirring is continued for about 6h until the swelling of the Rhodigel is complete.

## Claims

1. Compounds of formula 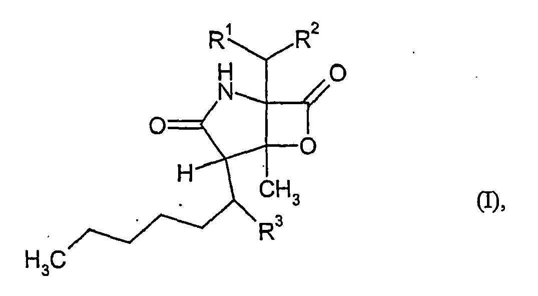 wherein
R¹ represents hydrogen, hydroxy or methylcarbonyloxy,
R² represents cyclohexyl or cyclohex-2-enyl, wherein cyclohexyl can be substituted with 0 to 2 hydroxy groups,
and
R³ represents hydrogen or hydroxy
and
their salts, solvates or solvates of the salts.

2. Compounds of formula (I) according to claim 1, with the formula 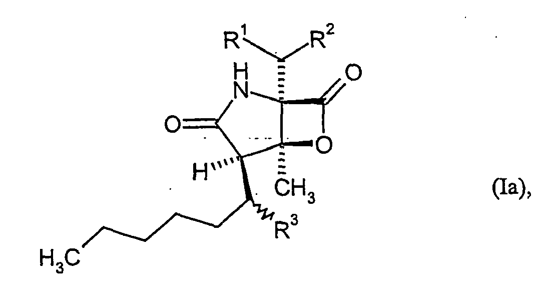 wherein
R¹, R² and R³ have the meaning described in claim 1,
and
their salts, solvates or solvates of the salts.

3. Compounds of formula (I) according to claim 1, such as
(1R,4R,5S)-1-[(S)-(1S)-2-cyclohexen-1-yl(hydroxy)methyl]-4-hexyl-5-methyl-6-oxa-2-azabicyclo[3.2.0]heptane-3,7-dione 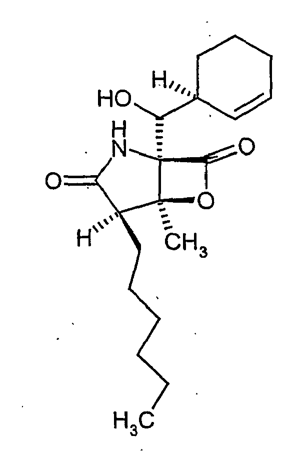 (1R,4R,5S)-1-[(S)-(1S)-2-cyclohexen-1-yl(hydroxy)methyl]-4-[1-hydroxy-hexyl]-5-methyl-6-oxa-2-azabicyclo[3.2.0]heptane-3,7-dione 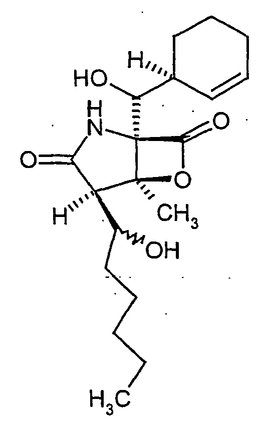 and
(1R,4R,5S)-1-[(1R)-2-cyclohexen-1-ylmethyl]-4-hexyl-5-methyl-6-oxa-2-azabicyclo-[3.2.0]heptane-3,7-dione

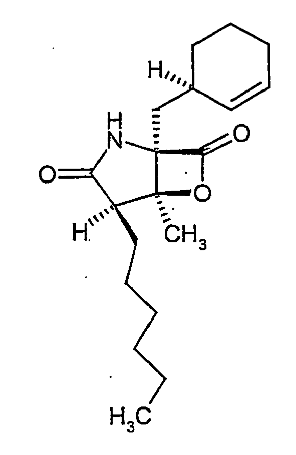

4. Compounds of formula 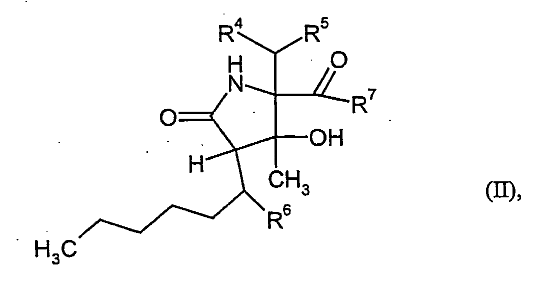 wherein
R⁴ represents hydrogen or hydroxy,
R⁵ represents cyclohexyl or cyclohex-2-enyl,
wherein cyclohexyl can be substituted with 0 to 2 hydroxy groups,
R⁶ represents hydrogen or hydroxy,
and
R⁷ represents hydroxy or
a substituent of the formula of the group consisting of 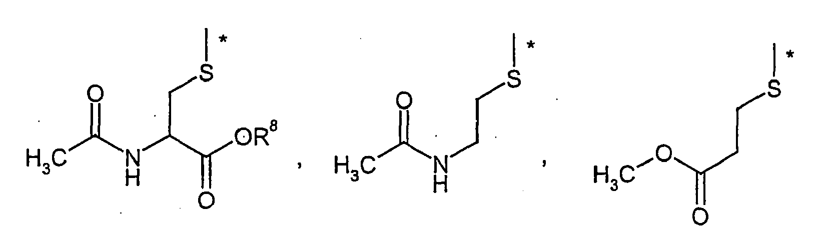 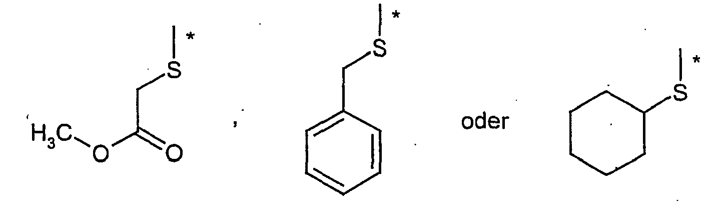 wherein
R⁸ represents hydrogen or methyl,
and
* represents the connection position to the molecule.
and
their salts, solvates or solvates of the salts.

5. Compounds of formula (II) according to claim 4, with the formula 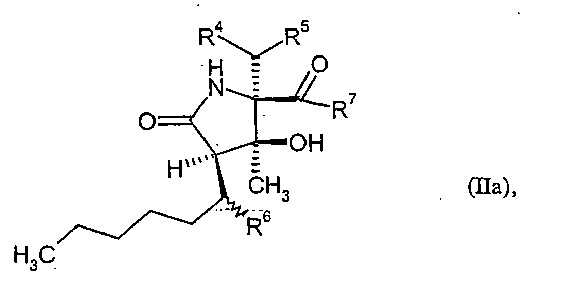 wherein
R⁴, R⁵, R⁶ and R⁷ have the meaning described in claim 4,
and
their salts, solvates or solvates of the salts.

6. Compounds of formula (II) according to claim 4, such as
(3S,4R)-2-[(S)-(1S)-2-cyclohexen-1-yl(hydroxy)methyl]-3-hydroxy-4-[1-hydroxyhexyl]-3-methyl-5-oxo-D-proline 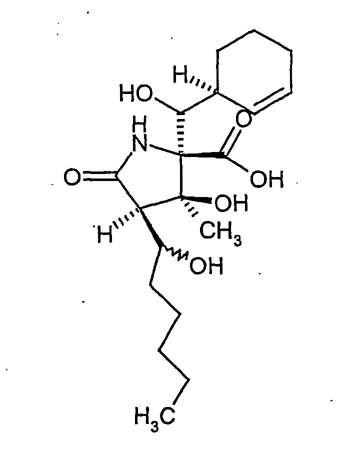 N-acetyl-S-({(2R,3S,4R)-2-[(S)-(1S)-2-cyclohexen-1-yl(hydroxy)methyl)-4-hexyl-3-hydroxy-3-methyl-5-oxo-2-pyrrolidinyl}carbonyl)cysteine 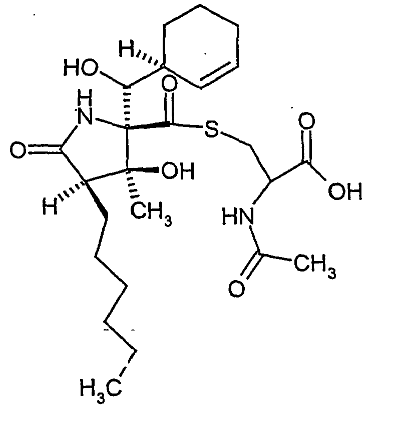 and
methyl-N-acetyl-S-({(2R,3S,4R)-2-[(S)-(1S)-2-cyclohexen-1-yl(hydroxy)-methyl]-4-hexyl-3-hydroxy-3-methyl-5-oxo-2-yrrolidinyl} carbonyl)cysteinate

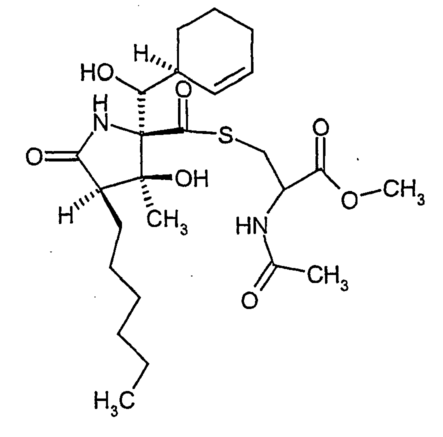

7. A process for synthesizing the compounds of general formula (I) and (Ia), wherein formula (I) contains the compounds of formula (Ib), (Ic), (Id) and (Ie), according to claim 1 or 2, and a process for synthesizing the compounds of general formula (II) and (IIa), wherein formula (II) contains the compounds of formula (IIb), (IIc) and (IId), according to claim 4 or 5, **characterized in that**
[A] the compounds of general formula 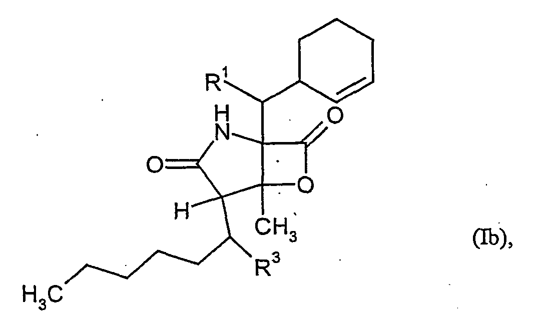 wherein
R¹ and R³ have the meaning described in claim 1,
are prepared via fermentation and isolation from an Actinomycete of the genus *Streptomyces* with SEQ ID NO: 1,
or
[B] the compounds of general formula 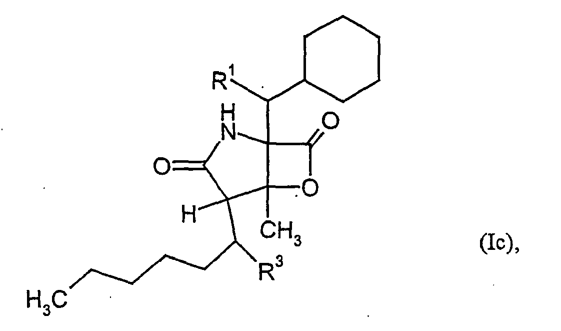 wherein
R¹ and R³ have the meaning described in claim 1,
are prepared via hydrogenation of the double bond in compounds of the formula (Ib),
or
[C] the compounds of general formula 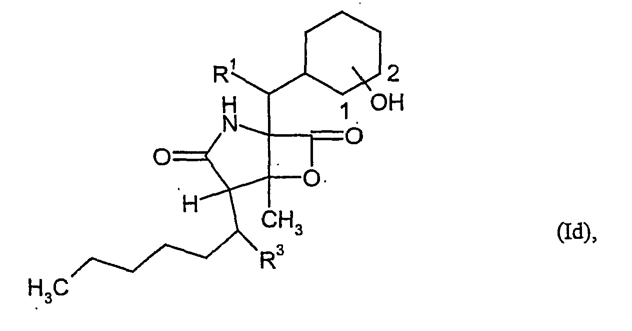 wherein
R¹ and R³ have the meaning described in claim 1, and
the hydroxy-group is attached onto carbon atom 1 or 2,
are prepared via hydration of the double bond in compounds of the Formula (Ib),
or
[D] the compounds of general formula 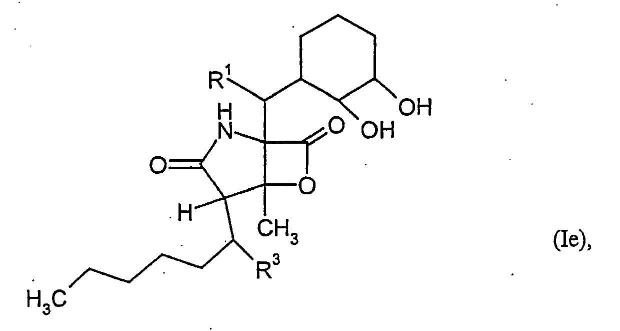 wherein
R¹ and R³ have the meaning described in claim 1,
are prepared via oxidation of the double bond in compounds of the formula (Ib),
or
[E] the compounds of general formula 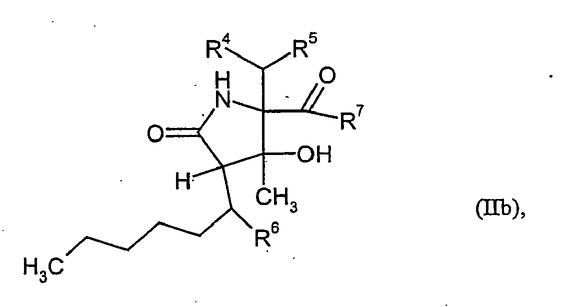 wherein
R⁴, R⁵ and R⁶ have the meaning described in claim 4, and
R⁷ represents hydroxy or a substituent of the formula 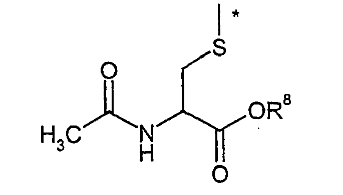 wherein
R⁸ has the meaning described in claim 4,
are prepared via fermentation and isolation from an Actinomycete of the genus *Streptomyces* with SEQ ID NO: 1,
or
[F] the compounds of general formula 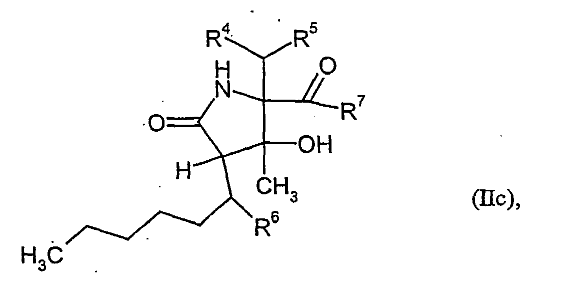 wherein
R⁴, R⁵ and R⁶ have the meaning described in claim 4, and
R⁷ represents a substituent of the formula of the group consisting of 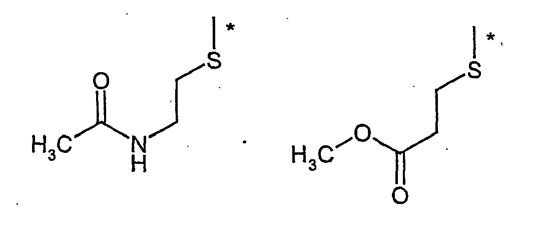 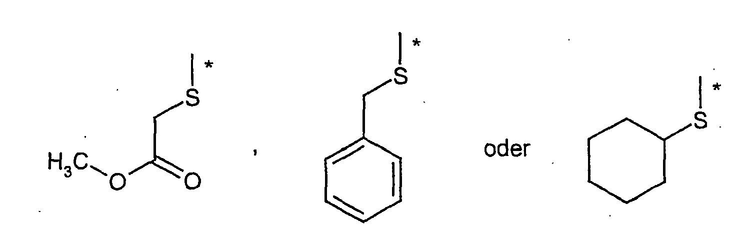 are prepared via reaction of the compounds of the formula 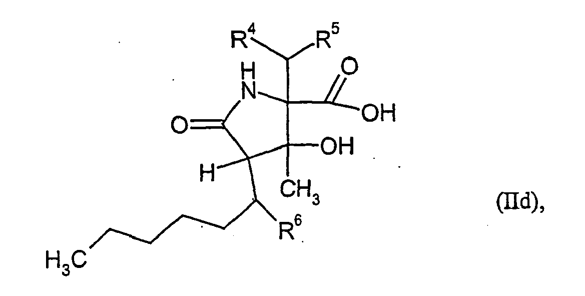 wherein
R⁴, R⁵ and R⁶ have the meaning described in claim 4,
with thioles.

8. The composition containing at least one compound of general formula (I), (Ia), (II) or (IIa) according to claim 1 to 6 and a pharmacologically acceptable diluent

9. A composition according to claim 8 for the treatment of acute and chronic inflammatory processes or cancer.

10. The process for the preparation of compositions according to claim 8 and 9 **characterized in that** the compounds of general formula (I), (Ia), (II) and (IIa) according to claim 1 to 6 together with customary auxiliaries are brought into a suitable application form.

11. Use of the compounds of general formula (I), (Ia), (II) or (IIa) according to claim 1 to 6 for the preparation of medicaments.

12. Use according to claim 11 for the preparation of medicaments for the treatment of acute and chronic inflammatory processes or cancer.

13. Compound according to claims 1-6 for the use in the control of acute and chronic inflammatory, processes in humans and animals.

14. Compound according to claims 1 to 6 for the use in the control of cancer processes in humans and animals.

15. Microorganism with the designation number DSM 15324 and the SEQ ID NO: 1.

16. Microorganism with the designation number DSM 15324 and the SEQ ID NO: 1 for the preparation of the compounds of formula (I), (Ia), (II) and (IIa).

## Patentansprüche

1. Verbindungen der Formel 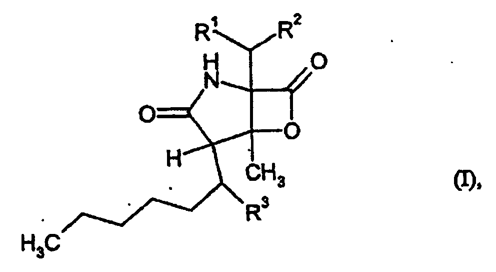 wobei
R¹ für Wasserstoff, Hydroxy oder Methylcarbonyloxy steht,
R² für Cyclohexyl oder Cyclohex-2-enyl steht, wobei Cyclohexyl durch 0 bis 2 Hydroxylgruppen substituiert sein kann,
und
R³ für Wasserstoff oder Hydroxy steht,
und
ihre Salze, Solvate und Solvate der Salze.

2. Verbindungen der Formel (I) nach Anspruch 1 mit der Formel 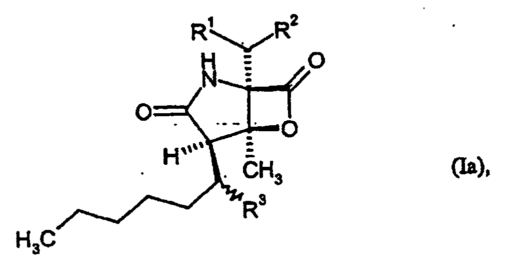 wobei
R¹, R² und R³ die in Anspruch 1 beschriebene Bedeutung haben,
und
ihre Salze, Solvate und Solvate der Salze.

3. Verbindungen der Formel (I) nach Anspruch 1 wie
(1R,4R,5S)-1-[(S)-(1S)-2-Cyclohexen-1-yl(hydroxy)methyl]-4-hexyl-5-methyl-6-oxa-2-azabicyclo[3.2.0]heptan-3,7-dion 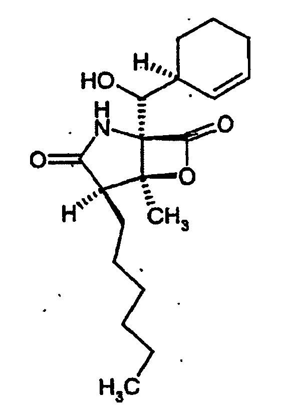 (1R,4R,5S)-1-[(S)-(1S)-2-Cyclohexen-1-yl(hydroxy)methyl]-4-[1-hydroxylhexyl]-5-methyl-6-oxa-2-azabicyclo[3.2.0]heptan-3,7-dion 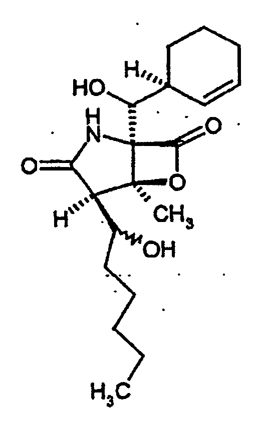 und
(lR,4R,SS)-1-[(lR)-2-Cyclohexen-1-ylmethyl]-4-hexyl-5-methyl-6-oxa-2-azabicyclo[3.2.0]heptan-3,7-dion

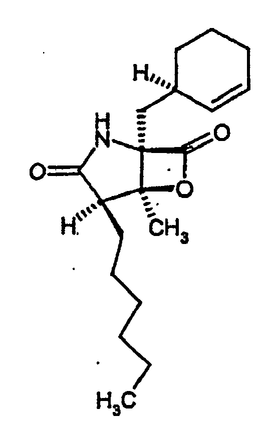

4. Verbindungen der Formel 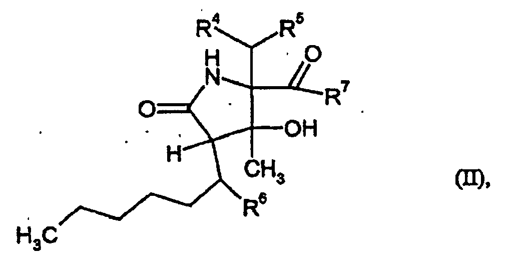 wobei
R⁴ für Wasserstoff oder Hydroxy steht,
R⁵ für Cyclohexyl oder Cyclohex-2-enyl steht,
wobei Cyclohexyl durch 0 bis 2 Hydroxylgruppen substituiert sein kann,
R⁶ für Wasserstoff oder Hydroxy steht,
und
R⁷ für Hydroxy oder einen Substituenten der Formel der aus 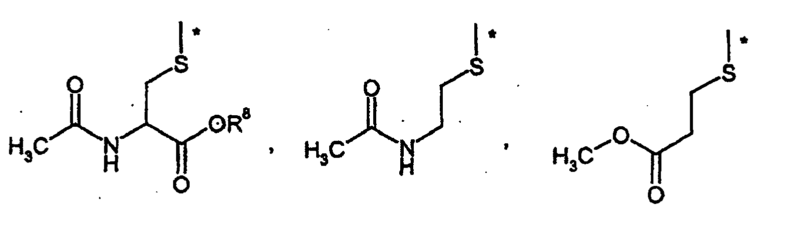 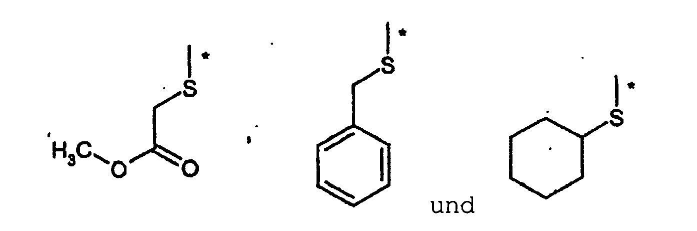 bestehenden Gruppe, wobei
R⁸ für Wasserstoff oder Methyl steht,
und
* für die Bindungsstelle an das Molekül steht,
und
ihre Salze, Solvate und Solvate der Salze.

5. Verbindungen der Formel (II) nach Anspruch 4 mit der Formel 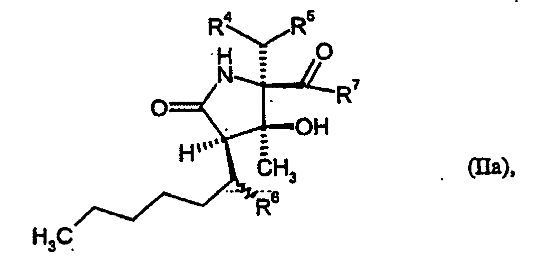 wobei
R⁴, R⁵, R⁶ und R⁷ die in Anspruch 4 beschriebene Bedeutung haben,
und
ihre Salze, Solvate und Solvate der Salze.

6. Verbindungen der Formel (II) nach Anspruch 4, wie
(3S,4R)-2-[(S)-(1S)-2-Cyclohexen-1-yl(hydroxy)methyl]-3-hydroxy-4-[1-hydroxyhexyl]-3-methyl-5-oxo-D-prolin 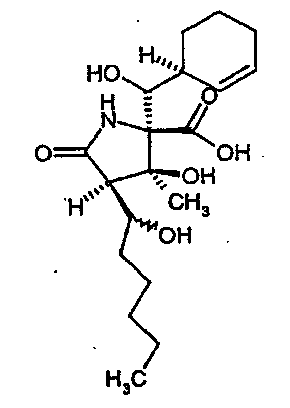 N-Acetyl-S-({(2R,3S,4R)-2-[(S)-(1S)-2-cyclohexen-1-yl(hydroxy)methyl]-4-hexyl-3-hydroxy-3-methyl-5-oxo-2-pyrrolidiniyl}carbonyl)cystein 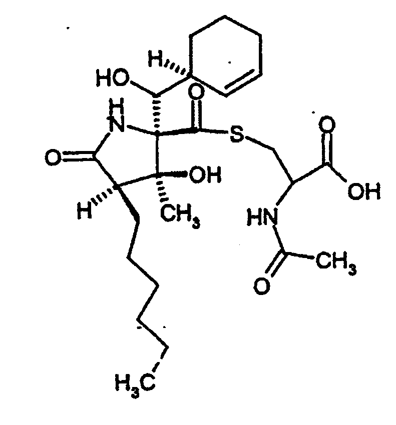 und
N-Acetyl-S-({(2R,3S,4R)-2-[(S)-(1S)-2-cyclohexen-1-yl(hydroxy)methyl]-4-hexyl-3-hydroxy-3-methyl-5-oxo-2-yrrolidinyl}carbonyl)cysteinmethylester

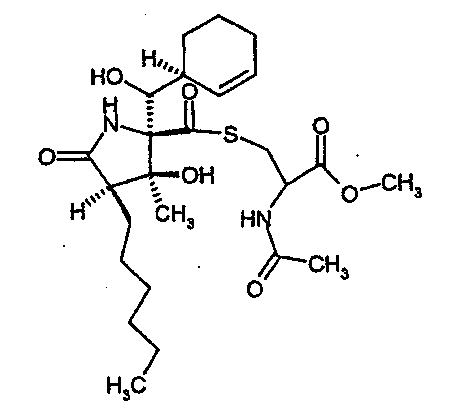

7. Verfahren zur Synthese der Verbindungen der allgemeinen Formeln (I) und (Ia), wobei die Formel (I) die Verbindungen der Formeln (Ib), (Ic), (Id) und (Ie) umfaßt, gemäß Anspruch 1 oder 2, und Verfahren zur Synthese der Verbindungen der allgemeinen Formeln (II) und (IIa), wobei die Formel (II) die Verbindungen der Formeln (IIb), (IIc) und (IId) umfaßt, gemäß Anspruch 4 oder 5, **dadurch gekennzeichnet, daß** man
[A] die Verbindungen der allgemeinen Formel 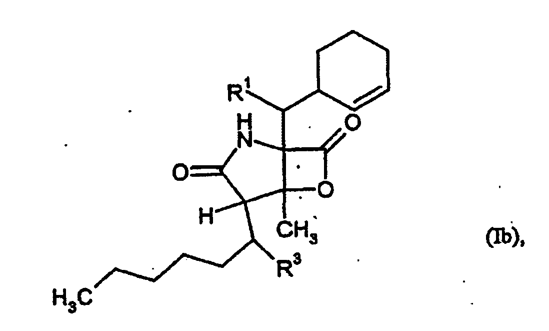 in welcher
R¹ und R³ die in Anspruch 1 beschriebene Bedeutung haben,
durch Fermentation und Isolation aus einem Actinomyceten der Gattung *Streptomyces* mit SEQ ID NO: 1: herstellt,
oder
[B] die Verbindungen der allgemeinen Formel 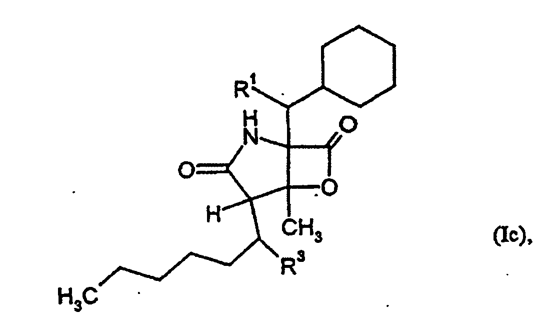 in welcher
R¹ und R³ die in Anspruch 1 beschriebene Bedeutung haben,
durch Hydrieren der Doppelbindung in Verbindungen der Formel (Ib) herstellt,
oder
[C] die Verbindungen der allgemeinen Formel 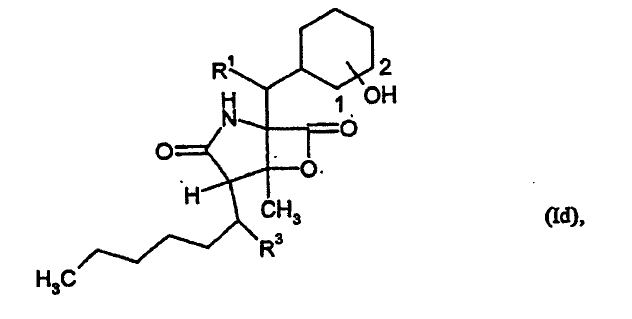 in welcher
R¹ und R³ die in Anspruch 1 beschriebene Bedeutung haben, und
die Hydroxylgruppe an das Kohlenstoffatom 1 oder 2 gebunden ist,
durch Hydratation der Doppelbindung in Verbindungen der Formel (Ib) herstellt,
oder
[D] die Verbindungen der allgemeinen Formel 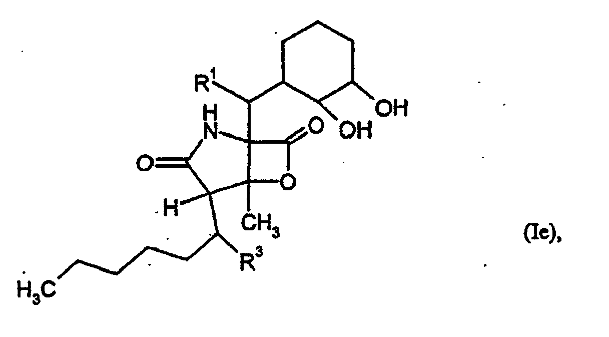 in welcher
R¹ und R³ die in Anspruch 1 beschriebene Bedeutung haben,
durch Oxidation der Doppelbindung in Verbindungen der Formel (Ib) herstellt,
oder
[E] die Verbindungen der allgemeinen Formel 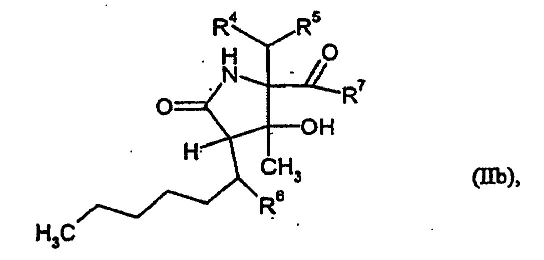 in welcher
R⁴, R⁵ und R⁶ die in Anspruch 4 beschriebene Bedeutung haben und
R⁷ für Hydroxy oder einen Substituenten der Formel 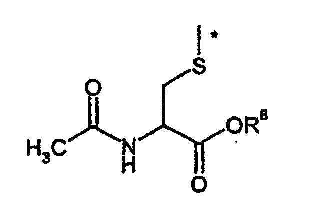 steht, wobei
R⁸ die in Anspruch 4 beschriebene Bedeutung hat,
durch Fermentation und Isolation aus einem Actinomyceten der Gattung *Streptomyces* mit SEQ ID NO: 1 herstellt,
oder
[F] die Verbindungen der allgemeinen Formel 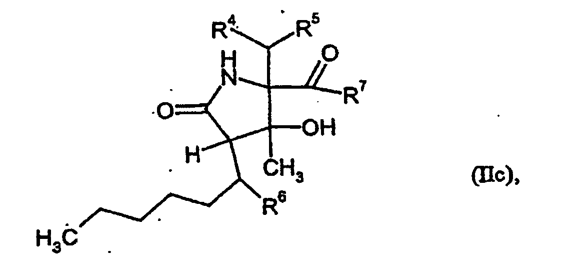 in welcher
R⁴, R⁵ und R⁶ die in Anspruch 4 beschriebene Bedeutung haben und
R⁷ für einen Substituenten der Formel der aus 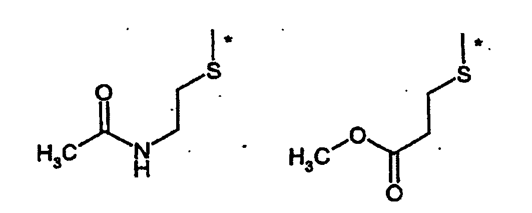 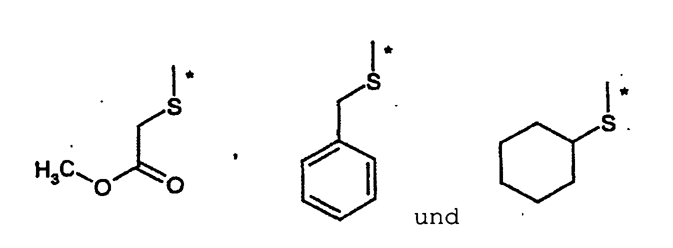 bestehenden Gruppe steht, durch die Umsetzung der Verbindungen der Formel 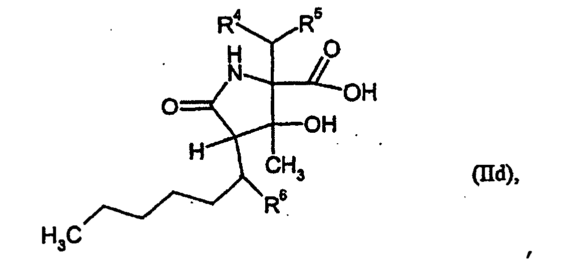 in welcher
R⁴, R⁵ und R⁶ die in Anspruch 4 beschriebene Bedeutung haben,
mit Thiolen herstellt.

8. Zusammensetzung, enthaltend wenigstens eine Verbindung der allgemeinen Formel (I), (Ia), (II) oder (IIa) nach Ansprüchen 1 bis 6 und ein pharmakologisch unbedenkliches Verdünnungsmittel.

9. Zusammensetzung nach Anspruch 8 zur Behandlung von akuten und chronischen entzündlichen Prozessen oder Krebs.

10. Verfahren zur Herstellung von Verbindungen nach Anspruch 8 und 9, **dadurch gekennzeichnet, daß** man die Verbindungen der allgemeinen Formel (I), (Ia), (II) und (IIa) nach Ansprüchen 1 bis 6 zusammen mit herkömmlichen Hilfsstoffen in eine geeignete Verabreichungsform bringt.

11. Verwendung der Verbindungen der allgemeinen Formel (I), (Ia), (II) oder (IIa) nach den Ansprüchen 1 bis 6 zur Herstellung von Medikamenten.

12. Verwendung nach Anspruch 11 zur Herstellung von Medikamenten zur Behandlung von akuten und chronischen entzündlichen Prozessen oder Krebs.

13. Verbindungen nach Ansprüchen 1 bis 6 zur Verwendung bei der Bekämpfung von akuten und chronischen entzündlichen Prozessen in Menschen und Tieren.

14. Verbindungen nach Ansprüchen 1 bis 6 zur Verwendung bei der Bekämpfung von Krebsprozessen in Menschen und Tieren.

15. Mikroorganismus mit der Bezeichnungsnummer DSM 15324 und SEQ ID NO: 1.

16. Mikroorganismus mit der Bezeichnungsnummer DSM 15324 und SEQ ID NO: 1 zur Herstellung der Verbindungen der Formeln (I), (Ia), (II) und (IIa).

## Revendications

1. Composés de formule 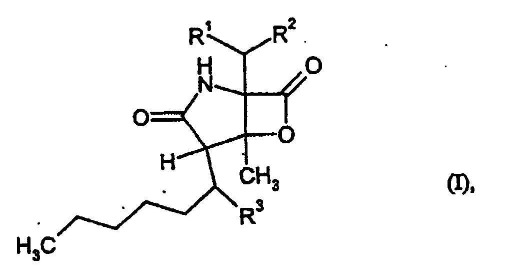 dans laquelle
R¹ représente hydrogène, hydroxy ou méthylcarbonyloxy,
R² représente cyclohexyle ou cyclohex-2-ényle, où cyclohexyle peut être substitué par 0 à 2 groupements hydroxy,
et
R³ représente hydrogène ou hydroxy,
et
leurs sels, solvates ou solvates des sels.

2. Composés de formule (I) selon la revendication 1, ayant la formule 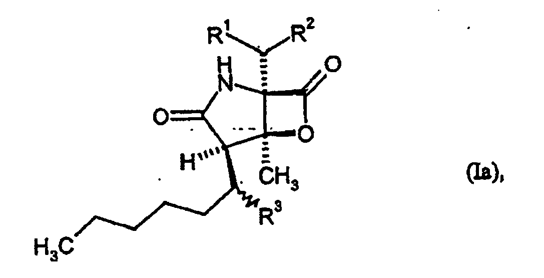 dans laquelle
R¹, R² et R³ ont les significations décrites dans la revendication 1,
et
leurs sels, solvates ou solvates des sels.

3. Composés de formule (I) selon la revendication 1, tels que
la (1R,4R,5S)-1-[(S)-(1S)-2-cyclohexén-1-yl(hydroxy)méthyl]-4-hexyl-5-méthyl-6-oxa-2-azabicyclo[3,2,0]heptane-3,7-dione 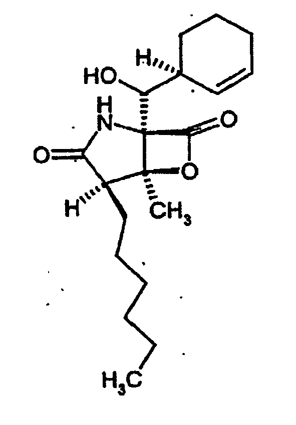 la (1R,4R,5S)-1-[(S)-(1S)-2-cyclohexén-1-yl(hydroxy)méthyl]-4-[1-hydroxylhexyl]-5-méthyl-6-oxa-2-azabicyclo[3,2,0]heptane-3,7-dione 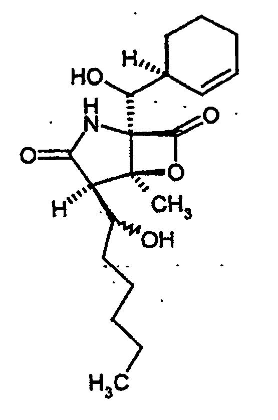 et
la (1R,4R,5S)-1-[(1R)-2-cyclohexén-1-ylméthyl]-4-hexyl-5-méthyl-6-oxa-2-azabicyclo[3,2,0]heptane-3,7-dione

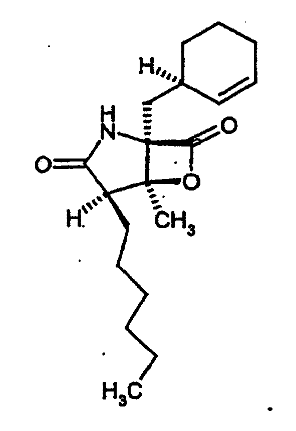

4. Composés de formule 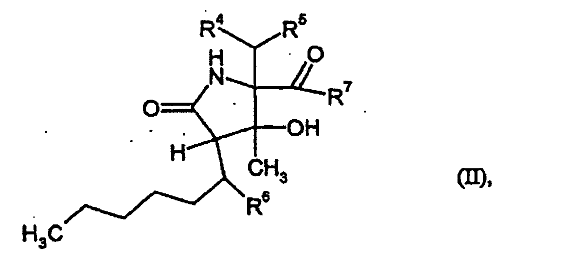 dans laquelle
R⁴ représente hydrogène ou hydroxy,
R⁵ représente cyclohexyle ou cyclohex-2-ényle,
où cyclohexyle peut être substitué par 0 à 2 groupements hydroxy,
R⁶ représente hydrogène ou hydroxy,
et
R⁷ représente hydroxy ou
un substituant de la formule du groupe constitué de 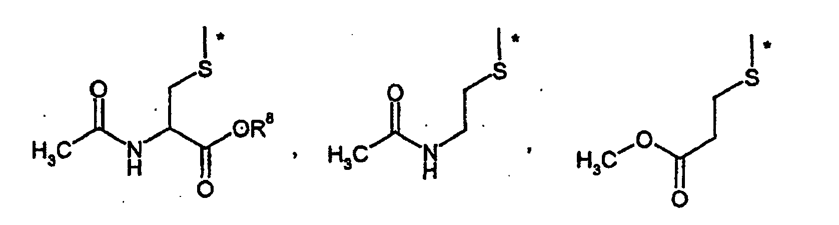 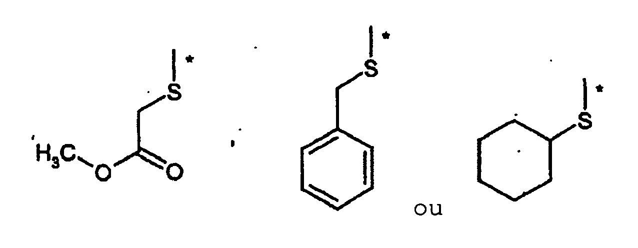 dans lequel
R⁸ représente hydrogène ou méthyle,
et
* représente la position de liaison à la molécule,
et
leurs sels, solvates ou solvates des sels.

5. Composés de formule (II) selon la revendication 4, ayant la formule 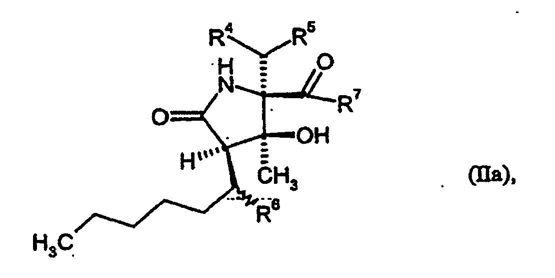 dans laquelle
R⁴, R⁵, R⁶ et R⁷ ont les significations décrites dans la revendication 4,
et
leurs sels, solvates ou solvates des sels.

6. Composés de formule (II) selon la revendication 4, tels que la (3S,4R)-2-[(S)-(1S)-2-cyclohexén-1-yl(hydroxy)méthyl]-3-hydroxy-4-[1-hydroxyhexyl]-3-méthyl-5-oxo-D-proline 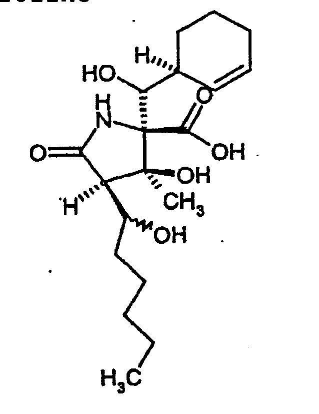 la N-acétyl-S-({(2R,3S,4R)-2-[(S)-(1S)-2-cyclohexén-1-yl(hydroxy)méthyl]-4-hexyl-3-hydroxy-3-méthyl-5-oxo-2-pyrrolidinyl}carbonyl)cystéine 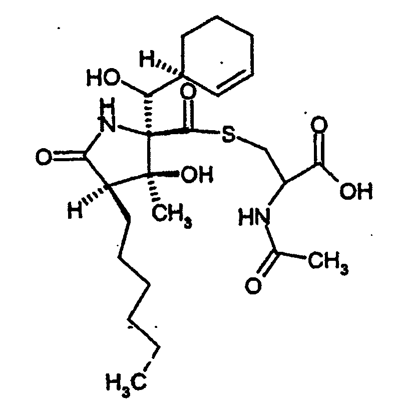 et
le N-acétyl-S-({(2R,3S,4R)-2-[(S)-(1S)-2-cyclohexén-1-yl(hydroxy)méthyl]-4-hexyl-3-hydroxy-3-méthyl-5-oxo-2-pyrrolidinyl}carbonyl)cystéinate de méthyle

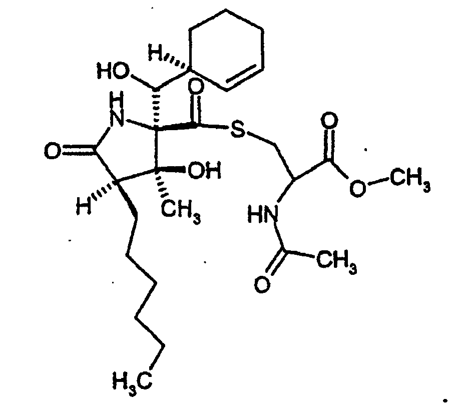

7. Procédé de synthèse des composés de formules générales (I) et (Ia), dans lequel la formule (I) contient les composés de formules (Ib), (Ic), (Id) et (Ie), selon la revendication 1 ou 2, et un procédé de synthèse des composés de formules générales (II) et (IIa), dans lequel la formule (II) contient les composés de formules (IIb), (IIc) et (IId), selon la revendication 4 ou 5, **caractérisés en ce que**
[A] les composés de formule générale 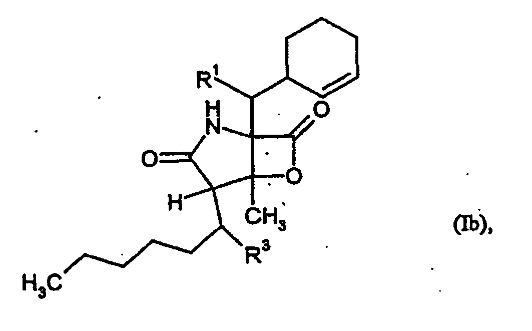 dans laquelle
R¹ et R³ ont les significations décrites dans la revendication 1,
sont préparés par fermentation et isolement à partir d'un actinomycète du genre *Streptomyces* avec SEQ ID NO: 1,
ou
[B] les composés de formule générale 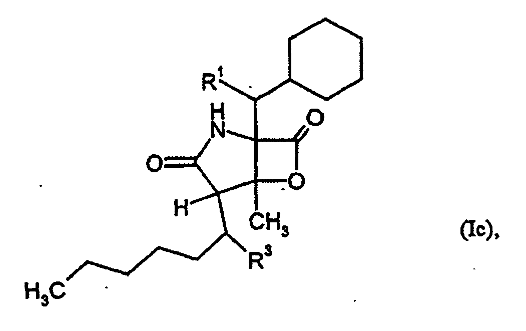 dans laquelle
R¹ et R³ ont les significations décrites dans la revendication 1,
sont préparés par hydrogénation de la double liaison dans les composés de formule (Ib),
ou
[C] les composés de formule générale 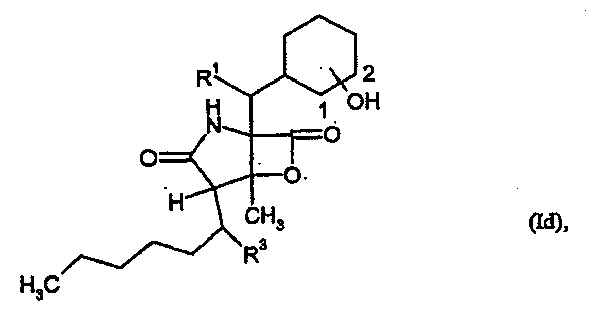 dans laquelle
R¹ et R³ ont les significations décrites dans la revendication 1, et
le groupement hydroxy est lié à l'atome de carbone 1 ou 2,
sont préparés par hydratation de la double liaison dans les composés de formule (Ib),
ou
[D] les composés de formule générale 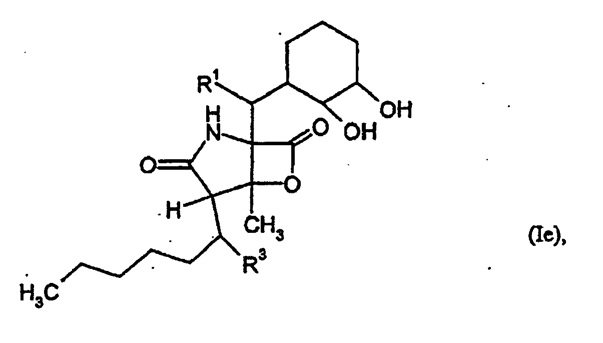 dans laquelle
R¹ et R³ ont les significations décrites dans la revendication 1,
sont préparés par oxydation de la double liaison dans les composés de formule (Ib),
ou
[E] les composés de formule générale 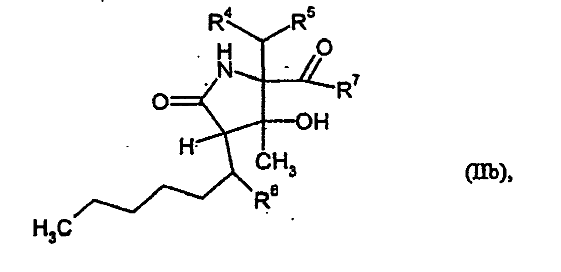 dans laquelle
R⁴, R⁵ et R⁶ ont les significations décrites dans la revendication 4, et
R⁷ représente hydroxy ou un substituant de formule 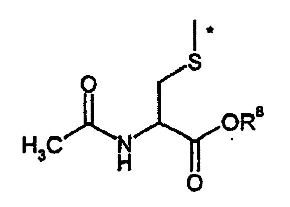 dans laquelle
R⁸ a la signification décrite dans la revendication 4,
sont préparés par fermentation et isolement à partir d'un actinomycète du genre *Streptomyces* avec SEQ ID NO: 1,
ou
[F] les composés de formule générale 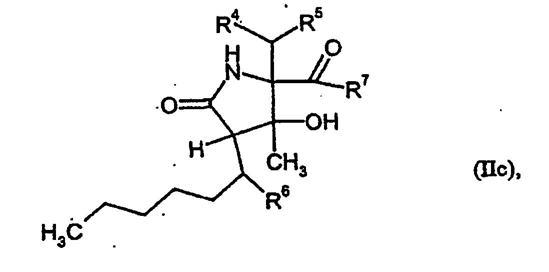 dans laquelle
R⁴, R⁵ et R⁶ ont les significations décrites dans la revendication 4, et
R⁷ représente un substituant de la formule du groupe constitué de 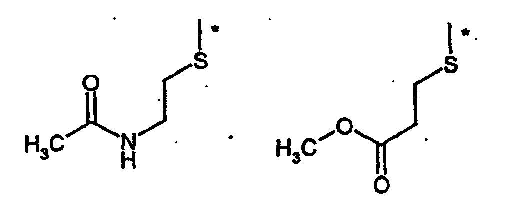 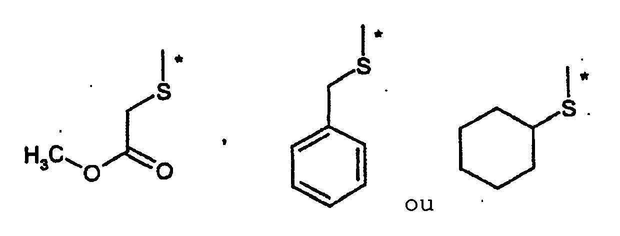 sont préparés par réaction des composés de formule 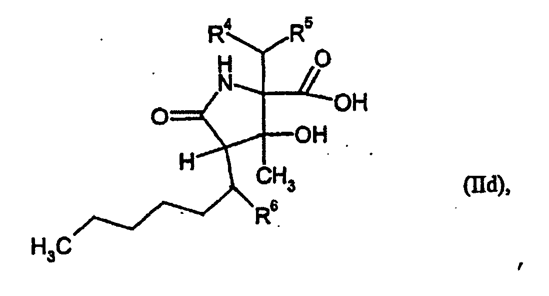 dans laquelle
R⁹, R⁵ et R⁶ ont les significations décrites dans la revendication 4,
avec des thioles.

8. Composition contenant au moins un composé de formule générale (I), (Ia), (II) ou (IIa) selon les revendications 1 à 6 et un diluant pharmacologiquement acceptable.

9. Composition selon la revendication 8, destinée au traitement de processus inflammatoires aigus et chroniques ou du cancer.

10. Procédé de préparation des compositions selon les revendications 8 et 9, **caractérisé en ce que** les composés de formules générales (I), (Ia), (II) et (IIa) selon les revendications 1 à 6, conjointement avec des auxiliaires usuels sont mis sous une forme d'application convenable.

11. Utilisation des composés de formule générale (I), (Ia), (II) ou (IIa) selon les revendications 1 à 6, pour la préparation de médicaments.

12. Utilisation selon la revendication 11, pour la préparation de médicaments destinés au traitement de processus inflammatoires aigus et chroniques ou du cancer.

13. Composé selon les revendications 1 à 6, destiné à être utilisé pour contrôler les processus inflammatoires aigus et chroniques chez l'homme et l'animal.

14. Composé selon les revendications 1 à 6, destiné à être utilisé pour contrôler les processus cancéreux chez l'homme et l'animal.

15. Microorganisme ayant le numéro de désignation DSM 15324 et SEQ ID NO: 1.

16. Microorganisme ayant le numéro de désignation DSM 15324 et SEQ ID NO: 1, destiné à la préparation des composés de formules (I), (Ia), (II) et (IIa).
